Europäisches Patentamt

European Patent Office

Office européen des brevets

(11) Publication number: **0 381 501 B1**

# EUROPEAN PATENT SPECIFICATION

(45) Date of publication of patent specification: **08.06.94**   (51) Int. Cl.5: **C12Q 1/68**, G01N 21/03

(21) Application number: **90301061.9**

(22) Date of filing: **01.02.90**

(54) **Containment cuvette for PCR and method of use.**

(30) Priority: **03.02.89 US 306735**
**17.04.89 US 339923**

(43) Date of publication of application:
**08.08.90 Bulletin  90/32**

(45) Publication of the grant of the patent:
**08.06.94 Bulletin  94/23**

(84) Designated Contracting States:
**BE CH DE DK FR GB IT LI LU NL SE**

(56) References cited:
EP-A- 0 237 362          EP-A- 0 281 201
EP-A- 0 318 255          FR-A- 2 612 295
US-A- 4 585 623          US-A- 4 690 801

(73) Proprietor: **EASTMAN KODAK COMPANY**
**343 State Street**
**Rochester New York 14650-2201(US)**

(72) Inventor: **Schnipelsky, Paul N., c/o EASTMAN KODAK Co.**
**Patent Dept.,**
**343 State Street**
**Rochester, NY 14650(US)**
Inventor: **Seaberg, Leonard J., c/o EASTMAN KODAK Co.**
**Patent Dept.,**

**343 State Street**
**Rochester, NY 14650(US)**
Inventor: **Wellman, Jeffrey A., c/o EASTMAN KODAK Co.**
**Patent Dept.,**
**343 State Street**
**Rochester, NY 14650(US)**
Inventor: **Hinckley, Charles C., c/o EASTMAN KODAK Co.**
**Patent Dept.,**
**343 State Street**
**Rochester, NY 14650(US)**
Inventor: **Donish, William H., c/o EASTMAN KODAK Co.**
**Patent Dept.,**
**343 State Street**
**Rochester, NY 14650(US)**
Inventor: **Findlay, John B., c/o EASTMAN KODAK Co.,**
**Patent Dept.,**
**343 State Street**
**Rochester, NY 14650(US)**

EP 0 381 501 B1

(74) Representative: **Phillips, Margaret Dawn et al
Kodak Limited
Patent Department
Headstone Drive
Harrow, Middlesex HA1 4TY (GB)**

## Description

This invention relates to cuvettes in which reactions are undertaken to amplify and detect nucleic acids, using polymerase chain reaction technology, without exposing the environment to amplified nucleic acid.

Polymerase chain reaction (PCR) technology permits nucleic acid material, such as DNA, often extracted from as little as a single cell, to be amplified to hundreds of millions of copies. This is important since prior to PCR technology it was virtually impossible to detect a single DNA strand. However, when a single DNA strand, such as the DNA of the human immunodeficiency virus (HIV, otherwise known to cause AIDS), is added to amplifying reagents which will amplify the chosen DNA, hundreds of millions of copies of that DNA can be obtained in a relatively short time. Technology further allows for the detection of the amplified nucleic acid material (DNA for example), using probes which hybridize to the amplified material of choice, such probes in turn either being immobilized or immobilizable to a solid support, such as a filter membrane, and/or being labeled for detection using enzymes or other moieties.

Conventionally, this has been done by amplifying the nucleic acid material in a stoppered plastic container until the desired number of copies have been formed. Then, the container is reopened, for example by unstoppering, and either the amplified copies are withdrawn and transferred to detection apparatus, or detecting reagents can be added to the container used for the amplification, so that detection is carried out in the same container.

It has been discovered that such a technique is unsatisfactory for convenient and widespread use of PCR technology, because aerosols are produced in the act of unstoppering the container and/or during transfer of fluids. Such aerosols contain a few molecules of the amplified nucleic acid material, e.g. DNA, become dispersed within the environment. Normally, such few molecules in the environment are not of great concern. However, only one DNA molecule is needed to contaminate other amplifying containers, which are as yet, unused. In particular, if an errant DNA molecule floats into or is carried, inadvertently, by an operator to another unused amplifying container, that one molecule is all that is needed to provide the DNA needed for the next amplification. Needless to say, if the point of the next test is to see if a particular DNA is present (e.g. for HIV), and it is detected only because of the errant DNA and not that of the patient, the test is ruined. Thus, the very power of DNA amplification becomes the source of potential ruin of the tests. As a matter of fact, an entire lab has been proven to be contaminated by the unstoppering of just a few containers in which the sample has already been amplified. Although such a problem might be avoidable by using highly skilled and trained personnel who painstakingly minimize the aerosols produced, the need for such labor makes the technology impractical for general use.

Thus, it has been a problem prior to this invention to provide apparatus and a method for amplifying and detecting nucleic acid material, without contaminating the surrounding environment.

Furthermore, efficient thermal transfer is required between sample and a heat source so that incubation of the sample can be achieved and so produce the desired amplification.

A further problem has been in automating the detection steps, and therefore minimizing the need for operator intervention. The need to transfer amplified nucleic acid material or to add detection reagents makes such automation difficult.

It is therefore an object of the present invention to overcome the problems mentioned above by providing apparatus and method for producing amplification and detection of nucleic acid material. The invention is based upon the realization that the contamination can be prevented by confining the amplifying reagents and amplified nucleic acid in the cuvette so that it is impossible for any amplified nucleic acid molecules to escape.

According to one aspect of the present invention, there is provided a disposable cuvette for carrying out amplification and detection of nucleic acid material comprising:

a first compartment;

a plurality of further compartments, at least one of the further compartments containing material to amplify and detect nucleic acid material;

fluid connection means for connecting at least one of the further compartments with the first compartment so that the material retained in the at least one further compartment is transferable to the first compartment when pressure is applied to the material retained in that compartment;

a detection site for immobilizing the nucleic acid material for detection after amplification; and

thermal transfer means for allowing active or passive cycling of material in the cuvette;

characterized in that the first compartment and the detection site are closed to fluid flow to locations outside of the cuvette so that aerosols are not emitted during amplification and detection.

By this arrangement, detection of amplified nucleic acid material occurs without contamination of other containers or apparatus by the amplified nucleic acid material.

According to a second aspect of the present invention, there is provided apparatus for amplifying and detecting DNA, comprising

a cuvette containing i) a plurality of compartments and means for interconnecting each of them to at least one other compartment, the compartments including a) at least one reaction compartment for amplifying DNA strands, b) at least one detection compartment for detecting amplified DNA and including a detection site, and c) means for delivering a detection material to amplified DNA strands;

ii) means for permitting active or passive cycling of the contents of the reaction compartment through a temperature range of from about 30°C to about 95°C; and

iii) liquid access means connected only to the at least one reaction compartment for allowing the injection into the reaction compartment of a sample DNA for amplifying;

characterized in that the cuvette further includes

iv) means for sealing the cuvette against passage of DNA after sample DNA is injected;

and the apparatus further includes means for moving at least the detection material and a DNA strand into the detection compartment and onto the detection site;

so that once a DNA sample is injected into the compartments and the access aperture is closed, the fluid contents of the compartments are contained against contact by the operator and environment during the entire amplification and detection reaction.

According to a third aspect of the present invention, there is provided a method for amplifying and detecting nucleic acid material in a closed cuvette comprising the steps of

a) injecting a sample of nucleic acid material into a cuvette which comprises a plurality of compartments including a reaction compartment wherein amplifying reagents are present, and a storage compartment for use with a detection material, at least one of the compartments including a detection site, and means for interconnecting the compartments to provide fluid transfer;

b) closing off permanently the portions of the cuvette containing the nucleic acid material to lock all nucleic acid into the cuvette;

c) amplifying the nucleic acid material by cycling the cuvette through temperature changes preselected to cause the reagents to be effective;

d) fluidly transferring amplified nucleic acid material from the reaction compartment to the detection site;

e) fluidly transferring detection material to the detection site; and

f) detecting the amplified nucleic acid material at the detection site with the detection material, the nucleic acid material remaining confined within the cuvette.

It is an advantageous feature of the invention that a cuvette is provided for amplifying nucleic acids which avoids the risk of contaminating the environment with amplified nucleic acid since it avoids reopening the area of the cuvette containing such nucleic acid.

It is a related advantageous feature of the invention that a cuvette is provided which can be used for such amplification by relatively unskilled labor.

It is another advantageous feature of the invention that such a cuvette is provided which is amenable to automated processing.

For a better understanding of the present invention, reference will now be made, by way of example only, to the accompanying drawings in which:-

Figure 1 is a plan view of a cuvette constructed in accordance with one embodiment of the present invention;

Figure 2 is a sectioned view taken generally along the line II-II of Figure 1;

Figure 3 is a sectioned view taken generally along the line of III-III of Figure 1;

Figure 4 is a fragmentary sectioned view taken along the line of IV-IV of Figure 1, but without the pipette;

Figure 5 is an enlarged, fragmentary sectioned view taken along the line V-V of Figure 1;

Figure 6 is a fragmentary plan view of a cuvette constructed in accordance with a second embodiment of the present invention;

Figure 7 is a plan view of a cuvette constructed in accordance with a third embodiment of the present invention;

Figure 8 is a sectioned view taken along the line VIII-VIII of Figure 7;

Figure 9 is a partially sectioned plan view of a cuvette constructed in accordance with a fourth embodiment of the present invention, the sectioned plane being generally taken along the line IX-IX of Figure 11;

Figures 10, 11, and 12 are sectioned views taken generally along the lines X-X, XI-XI, XII-XII, respectively, of Figure 9;

Figure 13 is a partially sectioned plan view of a cuvette constructed in accordance with a fifth embodiment of the present invention;

Figures 14 and 15 are fragmentary plan views partially in sectioned, of cuvettes constructed in accordance with two further embodiments of the present invention;

Figure 16 is a sectioned view taken generally along the line XVI-XVI of Figure 15;

Figure 17 is a fragmentary plan view partially in sectioned, of a cuvette constructed in accordance with another embodiment of the present invention;

Figure 18 is a sectioned view taken generally along the line XVIII-XVIII of Figure 17; and

Figure 19 is a sectioned view of a compartment of a cuvette constructed in accordance the present invention.

The invention is hereinafter described primarily with respect to the use of PCR technology to amplify and detect DNA, using particular preferred cuvette configurations. In addition, it is useful with any method of nucleic acid amplification, to amplify nucleic acid from any source, in any cuvette, so long as the apparatus and method prevent amplified nucleic acid from exiting the cuvette in any form. The nucleic acid can be obtained, for example, from plasmids or cloned DNA or RNA, or from natural DNA or RNA from any source, including bacteria, yeast, viruses, cells infected by viruses or bacteria, plants or animals. DNA or RNA may be extracted from blood or tissue materials. Another method of amplification called transcription-based amplification, which is different from PCR, can also benefit from the containment cuvette provided according to the present invention. Transcription-based amplification is described in Proc. Natl. Acad. Sci. USA, Volume 86, page 1173-1177, February, 1989 (Biochemistry).

Nucleic acid amplification generally proceeds via a particular protocol. One useful protocol is that set forth in US Patent Specification US-A-4683195. Briefly, that protocol features, in the case of DNA amplification, the steps of:

1) Obtaining a sample suspected of containing at least one specific nucleic acid sequence of interest;

2) Denaturing the ample to separate the strands;

3) Contacting the sample with primers, an extension enzyme such as polymerase and other amplification components useful to replicate the nucleic acid;

4) Repeating steps 2) and 3) as many times as necessary; and

5) Detecting the amplified DNA.

A preferred protocol within this class is as follows:

1) A complete DNA double helix is optionally chemically excised, using an appropriate restriction enzyme(s), to isolate the region of interest.

2) A solution of the isolated nucleic acid portion (here, DNA) and nucleotides is heated to and maintained at between 92° and 95°C for a length of time, e.g. no more than about 10 minutes, to denature the two nucleic acid strands; i.e. cause them to unwind and separate and form a template.

3) The solution is then cooled through a 30°C to 60°C zone, to cause a primer to anneal or "attach" to each of the two template strands. To make sure this happens, the solution is held at an appropriate temperature, such as about 55°C for about 15s, in an "incubation" zone.

4) The solution is then heated to and held at about 70°C, to cause an extension enzyme, preferably a thermostable polymerase enzyme, to extend the primers bound to the template strands by using the deoxyribonucleotides which are present.

5) The completed new pair of strands is heated again to between 92° and 95°C, for about 10 to 15s, to cause this pair to separate.

6) Steps 3) to 5) are then repeated a number of times until the appropriate number of strands are obtained. The more repetitions, the greater the number of multiples of the nucleic acid (here, DNA) which is produced. Preferably the desired concentration of nucleic acid is reached in a minimum amount of time, wherein each cycle takes less than one minute. However, as much as five minutes can be used for one cycle.

As used herein, the term "primer" refers to an oligonucleotide, whether naturally occurring or synthetically produced, which is capable of acting as a point of initiation of synthesis when placed under conditions in which synthesis of a primer extension product complementary to a nucleic acid strand is induced. Such conditions include the presence of nucleotides (such as the four standard deoxyribonucleotide triphosphates) and an agent for polymerization such as a DNA polymerase, and suitable temperature and pH. Generally, each primer used in this invention will have from 15 to 40 nucleotides, and preferably, it has from 20 to 25 nucleotides.

All of this is preferably done in a cuvette, using a cycling of temperature between about 30°C and about 95°C. The cuvette of the present invention provides a practical approach to allowing PCR technology to be practiced routinely by technicians and those of lesser skills, in an accurate fashion. For a complete understanding of the invention, further details of the PCR technology as it is practiced with this invention will be enumerated first.

Any DNA can be selectively replicated hundreds of millions of times. Selection of the appropriate primer nucleic acid strands insures that, under the best conditions, primarily the DNA of choice will replicate. Preferably, all primers are biotinylated when incorporated into the cuvette, to allow detection to proceed as described later. Heating of the target DNA now attached to a primer in the presence of an extension enzyme, produces a double strand which includes a copy of the DNA of choice. The new pair so formed is then separated by very short periods of high temperature denaturing, and the process repeated. This is all done in one reaction compartment by insuring that the primers, deoxyribonucleotides and extension enzymes are present when the sample is added, either as pre-incorporated reagents or reagents which are added with the DNA. If pre-incorporated, the reagents can be applied by spraying and drying, and can include a polymerase, salts, buffers, stabilizers, and the nucleotides needed for replication.

The polymerase enzyme is useful regardless of its source. Preferably, it is the polymerase naturally produced from Thermus aquaticus, hereinafter "TAQ", or any synthetic equivalent such as that which is genetically engineered, as described, for example, in published European patent application EP-A-0258017.

The presence of the enzymes emphasizes the need for rapid thermal cycling and short residence times at high temperatures. The denaturing temperature of between 92° and 95°C is close to the deactivation temperature of the enzymes, thus rendering unsatisfactory long heating periods.

Thereafter, the replicated DNA is identified, preferably by moving it to a detection compartment, to which suitable detection material is added or contained therein. In the prior art methods, such "movement" of the replicated DNA, and/or the addition of the reagents such as detection probes, has necessitated the reopening of the reaction compartment containing the DNA, creating the aerosol problem noted above. The detection involves the use of conventional materials capable of bonding via a complementary sequence of nucleotides to a replicated DNA strand. Such materials also include appropriate means which can be used to trap and hold the DNA at a detection site, such as in a detection compartment. Preferably, such appropriate means feature a membrane and/or a bead which is trapped.

Detection requires generally an immobilizing material and a signal generating material. Preferably, a primer used to replicate the DNA is alreayd biotinylated, so as to react with avidin which is attached to either the immobilizing material or the signal-generating material. If the avidin is attached to the immobilizing material (such as a bead), hereinafter, the "avidin-bead capture" method, then a detection probe is used with a nucleotide sequence which hybridizes with replicated primer and which either itself generates a signal (for example, by being radioactive), or reacts with a reagent which produces a signal. For example, the detection probe can be attached to any appropriate signal-generating moiety, preferably enzymes, for example, horseradish peroxidase, capable of reacting with a leuco dye to produce a detectable signal (e.g. a color change). The technology for attaching a signal generating moiety or an immobolizing material to a probe at the 3′ or 5′ end is known. For example in "Efficient Methods for Attachment of Thiol Specific Probes to the 3′ End of Synthetic Oligodeoxyribonucleotides", Vol. 15 of Nucleic Acids Research, p. 5303 (1987), the techniques useful for the 3′ end attachment are discussed. The articles discussing 5′ end attachment are legion, for which the following is only representative: "Introduction of 5′ Terminal Functional Groups. . . .", Vol. 164 of Analytical Biochemistry, p. 336 (1987). It will be readily apparent that either the 3′ or the 5′ end can be used to attach the signal-generating moiety on the immobilizing material.

Thus, as used herein the term "probe" refers to an oligonucleotide naturally occurring or synthetically produced, which does not act like a primer, but which is designed to be substantially complementary to one or more sequences of a nucleic acid so as to form a hybridized product. Further, a probe is generally designed for either "capture" or "detection" of the resulting hybridized product.

Alternatively, the detection probe and the immobilizing probe can be one and the same, attached at, say, just the 5′ end, using the techniques taught in the aforesaid Analytical Biochemistry article.

As noted above, the avidin can be attached to the signal-generating material, such as horseradish peroxidase, hereinafter the "oligo capture" method. In such a case, the immobilizing of the DNA is preferably achieved by an immobilizing probe, which is a nucleotide sequence which hybridizes with the replicated biotinylated primer, such sequence being attached to a polymer bead.

Thus, as used herein, "detection material" includes a probe on the replicated, biotinylated primer, which either itself generates a detectable signal or reacts with a reagent to produce a detectable signal. In the latter case, the detection material also includes such reagent.

The cuvette containing all detection materials, as well as the amplified DNA, can be agitated or shaken to promote mixing, and annealing of probes to the targeted DNA is achieved by conventional temperature cycling.

The hybridizing of the probes to the DNA can be done prior to or after transfer, if any, to the detection site.

Thereafter, all liquid is drawn off from the detection site. At this point, some of the detection material either is part of a replicated DNA strand or is hybridized to the DNA strand, and the DNA is captured by the surface of the membrane. Any DNA strand lacking the means for immobilizing it passes beyond the membrane.

The final step is to inject into the detection compartment a liquid containing a leuco dye or some other dye precursor capable of reacting with the detection material projecting from the DNA strands captured on the membrane. Useful leuco dyes include those disclosed in US Patent Specification US-A-4089747, preferably in combination with a solubilizing polymer such as poly(vinyl pyrrolidone). A preferred example of the dye is 2-(4-hydroxy-3,5-dimethoxyphenyl)-4,5-bis(4-methoxyphenyl)imidazole, since this gives about 1000 dye molecules per 1 molecule of horseradish peroxidase.

As noted, the DNA can attach to or be hybridized to a probe on a bead. The beads are selected to be trapped by the detection membrane. Useful material for such beads includes any polymer which has useful reactive groups for bonding to either avidin or to a probe which will hybridize to the DNA. Conventional covalent attachments of avidin via active halogen atoms, 2-substituted activated ethylsulfonyl, or vinylsulfonyl groups on such polymers are known. Thus, copolymers of m and p-(2-chloroethylsulfonylmethyl)styrene are useful, for example, for such beads.

There are two key aspects of this invention which make the aforedescribed procedure practical. The first is to use an efficient thermal transfer so that the contents of the reaction compartment are quickly heated and then quickly cooled. Means permitting either active or passive heating and cooling are useful, providing active or passive cycling. In particular, a Peltier device can be mounted in the reaction compartment to provide a heat transfer wall bordering the compartment. Preferably, however, the heat transfer is achieved by passive means, wherein the heat transfer material is a major wall surface of the reaction compartment. The heat source or heat sink is then supplied from an exterior source, most preferably on both sides of the cuvette.

The second key aspect is to construct the cuvette compartments to prevent amplified nucleic acid from escaping, namely, the compartments must be sealed against leakage to the environment once amplification occurs. A preferred construction is one in which the compartments have pre-incorporated all reagents before DNA is introduced, and locking means are used to lock the cuvette against leakage after DNA introduction. In such embodiments, means are provided for bringing about liquid communication between compartments within the closed cuvette, preferably using applied pressure, to obtain the necessary reactions.

A preferred thermal transfer mechanism will now be described. This mechanism transfers heat and/or cools a sample containing a nucleic acid material, such as DNA, to produce the amplification required for detection of that particular nucleic acid material. The material of the transfer wall is selected to provide a predetermined thermal path length and thermal resistance which will provide a high rate of thermal energy transfer. Most preferably, such path length is no greater than about 0.3mm, and the thermal resistance for a cross-sectional area of 1cm$^2$ is no greater than about 5.0°C/W. These properties are readily achieved by constructing the thermal transfer wall out of a plastic, or a laminate of plastic and metal, e.g. aluminium, which is about 0.05mm thick. The aluminium has a thermal resistance R, calculated as follows

$$R = \chi/(K \bullet A)$$

where

$\chi$ is the thickness of the aluminium,
K is the conductivity, and
A is the surface area.

The value of R is about 0.003°C/W. (This value can be compared with that for ordinary glass of the same thickness, glass having a thermal resistance of about 0.24°C/W.) The plastic, which is preferably a heat-sealable coated polyester such as poly-(ethylene terephthalate) coated on one or both sides with medium density polyethylene, has a thermal resistance of 1.06°C/W for a preferred thickness of about 0.005cm.

The thermal transfer wall can be secured to any of the other cuvette walls by any suitable means. One such means is a layer of a priming adhesive, which comprises, for example, a conventional high temperature acrylic adhesive, followed by a layer of conventional polyester adhesive. These adhesive layers can extend over the surface area of the thermal transfer wall, as such extensions can prevent the aluminium, if used, from interfering with reactions occurring within the cuvette. Alternatively, a plastic layer can cover the aluminium.

A cuvette constructed with such a thermal transfer wall has been found to produce a thermal time constant, $\tau$, for a volume of liquid of about 200$\mu$l, which is no greater than about 10s. Most preferred are those values for which $\tau$ is of the order of 3 to 8s. For example, when such a cuvette, filled with water, is heated along the exterior of the thermal transfer wall, and its temperature is measured at point inside the reaction compartment on the other side of that wall, a thermal response curve can be generated from 28°C to a final temperature of 103.9°C. The time taken for the liquid therein to reach a temperature of 76°C (that is, 63% of the difference between the final and initial temperatures (103.9 - 28)) is the value of $\tau$. This derives (approximately) from the well-known thermal response equation:

$$T(t) = T_f + (T_i - T_f) \cdot e^{-t/\tau} \quad (1)$$

where

> $T(t)$      is the temperature at time t, and
>
> $T_f$ and $T_i$      are the final and initial temperatures respectively.

Thus, if the time interval, t, in question equals $\tau$, then $e^{-t/\tau} = e^{-1} \approx 0.37$. In such a case, $T(t)$ (at t $= \tau$) is the temperature which is equal to the sum of the initial temperature, $T_i$, plus 63% of $(T_f - T_i)$. From such values, $\tau$ for the liquid in the cuvette turns out to be about 3.5s. For the preferred configurations, using an intervening layer between the aluminium and the liquid in the reaction compartment, $\tau$, the thermal time constant, is still no greater than about 10s when the liquid in the cuvette is water.

Alternatively, the heat source can be a defocused laser. The use of just a clear polyester layer as the thermal transfer wall is preferred in such a case, and a dye is incorporated into the reaction compartment, having an absorption wavelength appropriate to the laser.

As mentioned previously, one problem which is overcome by the present invention, is contamination of the environment due to the aerosol effect of opening or de-stoppering a cuvette in which amplification has taken place. In order to avoid contamination of the environment, the amplified DNA must be locked within the cuvette. This means that reagent needed for amplifications, that is, the primer strands, deoxyribonucleotides and the extension enzymes, are either pre-incorporated into the cuvette prior to addition of the sample of nucleic acid material, or they are added with the sample. Most preferably, the detection material is pre-incorporated prior to addition of the sample, so that after sample addition and prior to amplification, the cuvette is locked shut against leakage, there being no further access required. Alternatively, however, the cuvette can be constructed to allow the detection material to be added to storage compartments post amplification, subject to these provisions:

> 1) the storage compartment(s) to which they are added must be separate from the reaction compartment used for amplification, and
>
> 2) there must be provided means such as one-way check valves which allow such storage compartments to feed reagent to the amplified nucleic acid, but not amplified nucleic acid to the storage compartments.

In order to achieve this, the cuvette is provided with means for providing communication between the storage compartments and the detection compartment. In one embodiment, such communications means include means such as pressurizing members for moving the reagents from the storage compartment to the detection site, for example, a separate detection compartment. Alternatively, and most preferably, the pressurizing means are exterior to the cuvette, and the walls of the cuvette are flexible enough to transmit pressure from the exterior to the interior, thus pressurizing and moving the reagents within the cuvette. Any external pressure source can be used, e.g. a pressure roller or a piston from an air cylinder.

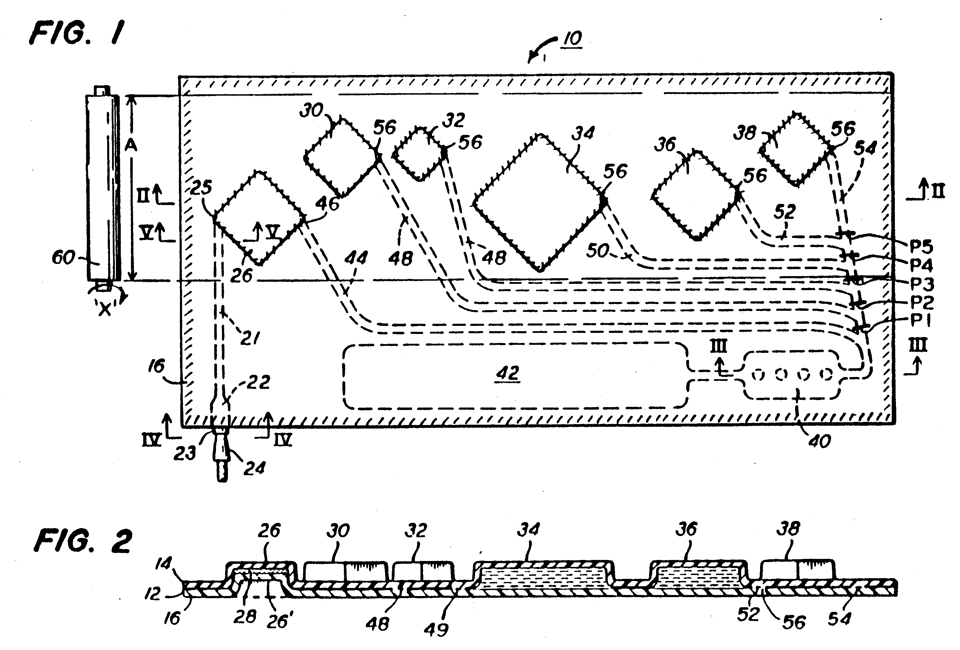

Figures 1 to 5 illustrate one embodiment of a cuvette 10. The cuvette 10 has flexible compartments which can cooperate with external pressurizing means 60, to provide the total apparatus of the present invention. More particularly, the cuvette 10 comprises two relatively thin sheets 12, 14 which are formed by molding them with pockets or compartments 26, 30, 32, 34, 36, 38, 40, 42, and connecting passageways 44, 48, 50, 52, 54 which protrude from the plane in which the sheets 12, 14 contact one another (see Figure 2). The sheets 12, 14 are secured together at least along their outer periphery 16, and preferably at all points surrounding compartments or passageways, such as by heat- and/or ultrasonic pressure-sealing. A heat-activatable adhesive such as ethylene vinyl acetate is useful for such joining operation.

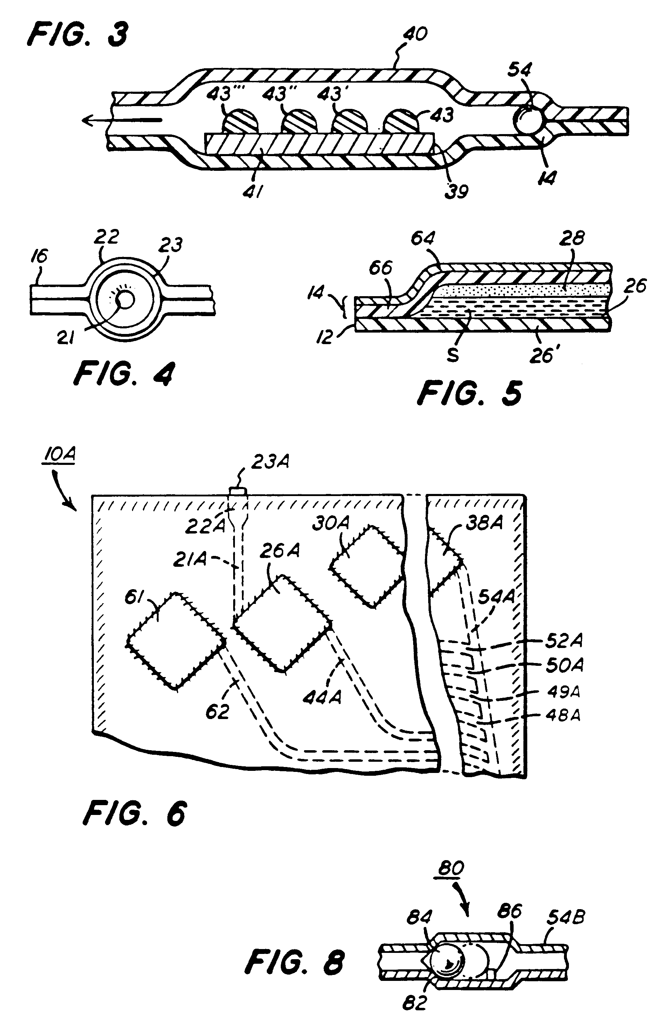

A liquid injection aperture 22 is the exception to the sealed periphery 16, for use with a mating pipette 24. Such aperture 22 optionally includes a rigid rim 23 extending into it within which a pipette 24 seats as is shown in Figure 4.

Compartment 26 is a reaction compartment in which amplifying reagents 28 in liquid or dried form may optionally be pre-incorporated as shown in Figure 2.

Compartment 30 is a storage compartment for a first wash of the process and contains wash water as a pre-incorporated reagent.

Compartment 32 is another storage compartment and contains at least one of the detection materials pre-incorporated therein, namely a biotinylated probe having at one end a complementary nucleotide for attachment to the amplified DNA. Preferably a signal generating moiety, for example, avidin bound to the horseradish peroxidase as discussed above is also pre-incorporated into storage compartment 32.

Compartment 34 is a storage compartment for a second wash. Preferably, this compartment has a much larger volume than that of compartment 32.

Compartment 36 has the remaining detection reagents pre-incorporated therein. These are a peroxide and a leuco dye, for example 2-(4-hydroxy-3,5-dimethoxyphenyl)-4,5-bis(4-methoxyphenyl)-imidazole, preferably in combination with poly(viny pyrrolidone) as a stabilizer.

Compartment 38 has a stop solution pre-incorporated therein which prevent too much leuco dye from converting to the dye. This stop solution may be, for example, a solution of sodium azide

Compartment 40 contains the detection site (39) for the cuvette of this embodiment, and will be described later. Compartment 42 is the waste compartment, which is, preferably initially deflated to provide for expansion as liquid is forced into it. Compartment 26 connects to compartment 40 via passageway 44. Optionally, a one-way check valve (not shown) can be included in passageway 44 to prevent waste liquid from backwashing into compartment 40, thus creating undesirable background color.

Passageway 21 connects injection aperture 22 with reaction compartment 26, and passageway 44 connects reaction compartment 26 with detection compartment 40, except that a temporary seal is provided at 46 to keep introduced DNA in compartment 26 until pressure is generated by roller 60. Compartment 30, compartment 32, compartment 34, compartment 36, and compartment 38 are all connected with detection compartment 40 by means of respective passageways 48, 49, 50, 52, and 54. Each compartment is preferably provided with a temporary seal 56 for interrupting flow out of the respective compartment until roller 60 breaks the seal. The position of the seal 56 is is shown in Figure 2. Passageway 54 serves as the trunk line to which the other passageways 48, 49, 50 and 52 are joined.

The compartments are deliberately positioned, as shown in Figure 1, so that each one will empty into compartment 40 in the proper sequence as roller 60 advances along path A in the direction of arrows 'X'. The sequence is as follows:

(1) the amplified DNA (from compartment 26) is pushed into compartment 40;
(2) the first wash (from compartment 30);
(3) the detection probe from compartment 32;
(4) the second wash (from compartment 34);
(5) the leuco dye solution (from compartment 36); and
(6) the stop solution (from compartment 38).

In some cases, the development of the dye from the leuco dye needs to be carried out in the dark, for example, if the dye fades readily in light.

The respective passageways are also preferably constructed so as to be squeezed by the roller, in particular, they are constructed to always form an angle to arrows X that is less than a right angle, within path A of roller 60. If they were to form a right angle, the roller would tend to jump over the passageway, rather than squeeze it.

It is not essential that both sheets 12 and 14 be collapsible by roller 60. Only one of the sheets 12, 14 needs to be collapsible, for example, under a pressure of at least 170g/cm². Pressures as high as 1500g/cm² are also useful. Therefore, sheet 12 can comprise a collapsible, relatively flexible plastic such as a heat-sealable polyester, for example Scotchpak™ brand heat-sealable film no.229 made by 3M, whereas sheet 14 can be less flexible and less collapsible, or it can be of the same flexibility as sheet 12.

Sheet 14 may comprise a laminate at least for compartment 26. The laminate comprises an aluminium foil 64 on the outside, and a polymer layer 66 on the inside, as shown in Figure 5. Preferably the polymer layer 66 is a layer of polyester, similar to sheet 12.

The aluminium foil preferably has a thickness of between about 0.0013cm and about 0.026cm, and most preferably about 0.005cm. Layer 66 has a thickness of between about 0.0013cm and about 0.03cm, and most preferably about 0.005cm. Even with layer 66 present, the thermal path length of compartment 26 is no more than about 0.3mm and the thermal resistance does not exceed about 5.0°C/watt.

The advantage of the laminate construction over a single sheet of plastic is that, once the compartment is crushed by the roller, the aluminium resists reinflation such as could allow backwashing to occur from liquids under pressure downstream. For this reason, sheet 14 preferably constructed as a laminate for the entire length of cuvette 10.

It is preferred that a liquid, when ejected from its compartment, will not backwash up to the passageway used to empty another compartment that is further downstream. To this end, as roller 60 advances from left to right, as shown in Figure 1, pinching means can be used to pinch off the passageways as follows:

As roller 60 moves across compartment 26, pinching is done at point $P_1$. As it moves across compartment 30, pinching occurs at point $P_2$, and likewise at point $P_3$ for compartment 32, point $P_4$ for compartment 34 and point $P_5$ for compartment 36.

Alternatively, a prewash compartment can be included, as shown in a second embodiment of a cuvette 10A illustrated in Figure 6. This ensures that all the exit passageways are first filled with water, so that upstream compartments will not backwash into the passageways for downstream compartments. Parts similar to those previously described, bear the same reference numeral to which the distinguishing suffix "A" has been appended.

In Figure 6, the very first compartment to be encountered by roller 60 can be storage compartment 61 containing wash water which empties via passageway 62 to trunk line 54A. The rest of the passageways 44A, 48A, 49A, 50A and 52A all connect from their respective compartments as described previously. The injection passageway 21A and aperture 22A for compartment 26A are moved to the opposite edge of cuvette 10A, because of compartment 61. The function of compartment 61 and passageway 62 is to flood all passageways of all the compartments with wash water, when the roller flattens that compartment. Thereafter, when each successive compartment is flattened by the roller, there will be no opportunity for, say, the amplified DNA of compartment 26A to push into any of passageways 48A, 49A, 50A or 52A, because of the water already there. Such water will not adversely affect the transmission of each compartment's contents to the detection compartment 40.

Compartment 61 can provide an additional advantage of allowing re-constitution of dried reagents stored in compartments 32A, 36A and 38A. If the light heat seal used (as hereinafter described) to close off the exit of each compartment to its respective passageway is omitted and dried reagents are stored in their respective compartments, when compartment 61 is pressurized by the roller 60, water from compartment 61 will flood the cuvette 10A and reconstitute the dried reagents stored in compartments 32A, 36A, and 38A. 0ptionally, this can be aided by shaking the cuvette. The reconstitution step can occur before or after sample injection into the reaction compartment or amplification within the reaction compartment.

The two embodiments described above feature sequential pressurization of each of the compartments. In addition, simultaneous pressurization of all the liquid containing compartments can be used, provided that pinch points $P_1$, $P_2$, $P_3$, $P_4$ and $P_5$, in Figure 1, are also used. If pressure is applied to all of $P_1$, $P_2$, $P_3$, $P_4$ and $P_5$ to close off the exit passageways except for passageway 44, pressure can be simultaneously applied (for example, by appropriately placed air pistons) to all of compartments 26, 30, 32, 34, 36 and 38. However, since only passageway 44 is unblocked, only the amplified DNA will be transferred to the detection compartment 40. Thereafter, the pinch points are released in sequence. As pinch point $P_1$ is released, wash water is allowed into passageway 48, and as pinch point $P_2$ is released the detection probe from compartment 32 is allowed into passageway 49, and so forth until pinch point $P_5$ is finally released, allowing the stop solution to enter the detection compartment via passageway 54. Care should be taken to ensure that the exerted pressure is less than the pressure required to burst the seams confining the liquid in the respective compartments and passageways.

Detection compartment 40, as shown in Figures 1 and 3, is a flow-by compartment comprising a detecting member 39 which comprises a supporting sheet 41 on which are disposed piles 43, 43', 43", and 43‴. If the oligo capture method is being used, then each pile 43, 43', 43", and 43‴ comprises polymer beads to which the detection probes noted above are immovably attached. The detection probes are constructed to hybridize with the DNA so that it can be detected. Most preferably, each bead has a different detection probe for a different DNA, so that if enough different piles of beads are present, for example, 8 to 10, tissue typing can be carried out on the basis of seeing which beads change color due to the dye from compartment 36. It is conventional to use latex beads in such an application.

Sheet 41 is made from a material which will bond to the pile of beads, keeping them in place once they have been deposited and dried during manufacturing. Useful examples of materials from which sheet 41 could be made include nitrocellulose, porous nylon membranes such as those manufactured by Pall Corp., and most preferably a paper coated with latex. An example of such a latex coated paper comprises a paper weighing about $54g/m^2$ and having a thickness of about 0.6mm, with a neutral internal sizing, made from about 80% hardwood, can be surface sized and then coated with a latex coating at an average of about $7g/m^2$. The coating is composed of a conventional industrial grade latex, NaOH at 20 weight %, TSPP as a dispersing agent, opacifying agents such as silicon dioxide and titanium dioxide, hydrasperse clay, and the rest distilled water.

The sheets 12 and 14 are prepared and assembled as follows: sheet 14 is premolded with the compartment indentations formed as shown in Figures 1 and 2. With sheet 14 turned upside down, the indentations forming cups, reagents can be then applied, for example dried reagents 28 and the liquids that go into compartments 30, 32, 34, 36 and 38. Next, sheet 12, now an "upper" sheet, is superposed while essentially flat, except for the mating depression shown at 26' in Figure 2. The two sheets are then lightly anchored together around the perimeter of each compartment as shown by hatching lines in Figure 1, except at the junction 25 of passageway 21 with compartment 26. For example, a light heat-sealing will bond the two plastic sheets together at these portions, including the outlet of each compartment to its respective exit passageway to provide the temporary seal 56. Each temporary seal 56 prevents liquid proceeding out of a compartment once it is introduced. However, this seal 56 is overcome when roller 60 is applied. (Such temporary seals appear as a broken line at the cross-section of passageways 48, 49, 52 and 54 as shown in Figure 2, and represent a site of separation when liquid is forced out of the compartments.)

Thereafter, a heavy seal, e.g., a heat seal, is applied around the circumference of each compartment and its exit passageway, but not across the junction of the passageway to the compartment, that is the operation of the temporary seal 56 is not affected. The heavy seal is also applied to the outer periphery 16. The sealing around compartments ensures that liquid pressed out of the compartments will flow only along the respective passageways and not elsewhere between sheets 12 and 14.

When cuvette 10 is used, the patient sample S is injected into compartment 26, as shown in more detail in Figure 5, via a pipette 24, at aperture 22. This causes depressed portion 26' to "pop out" enough to become about flush with the rest of sheet 12, shown in phantom in Figure 2 and in solid line in Figure 5.

Alternatively, portion 26' can be forced to "pop out" beyond the plane of the rest of sheet 12, to form an opposite blister 26", as shown in Figure 19. Furthermore, as shown in Figure 19, sheets 12 and 14 need not have any metallic component or layer, and can consist entirely of plastic, that is, a plastic sheet alone can provide sufficient rates of thermal transfer.

It is essential that aperture 22, as shown in Figures 1 and 4, be closable after pipette 24 is withdrawn, and prior to the amplification step. This can be accomplished by heat-sealing the aperture closed, or by stoppering the aperture in a suitable fashion, such as by heat-sealing strips 12 and 14 with the heavy seal, or constructing rim 23 with a one-way valve, not shown. If aperture 22 is to be heat-sealed, preferably rim 23 is omitted and pipette 24 is simply pushed directly into the aperture. Whatever mode of closure is used, it should be effective to resist any pressure build-up during PCR amplification or during liquid transfer. However, heat-sealing is the preferred method.

As noted above, heating and cooling to provide the needed thermal PCR cycling preferably occurs by heating one or both strips 12 and 14 at compartment 26.

When the amplified DNA enters compartment 40, it is retained there briefly while heat is applied through strip 14 to effect hybridization. Preferably, strip 14 is transparent at compartment 40 to allow transmission of radiation of suitable wavelength, e.g., visible wavelengths to allow examination of the contents. Compartment 42 can expand to accommodate the liquid influx as well as air influx, since it is preferably deflated prior to use. Alternatively, the instruments used to process the cuvette can include a vacuum plate which pulls compartment 42 out to its inflated shape when the waste volume is needed.

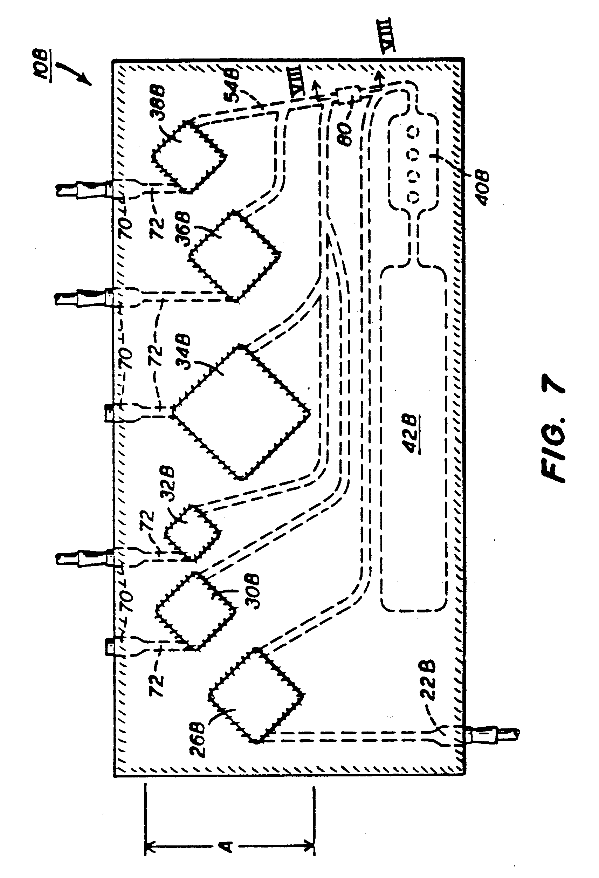

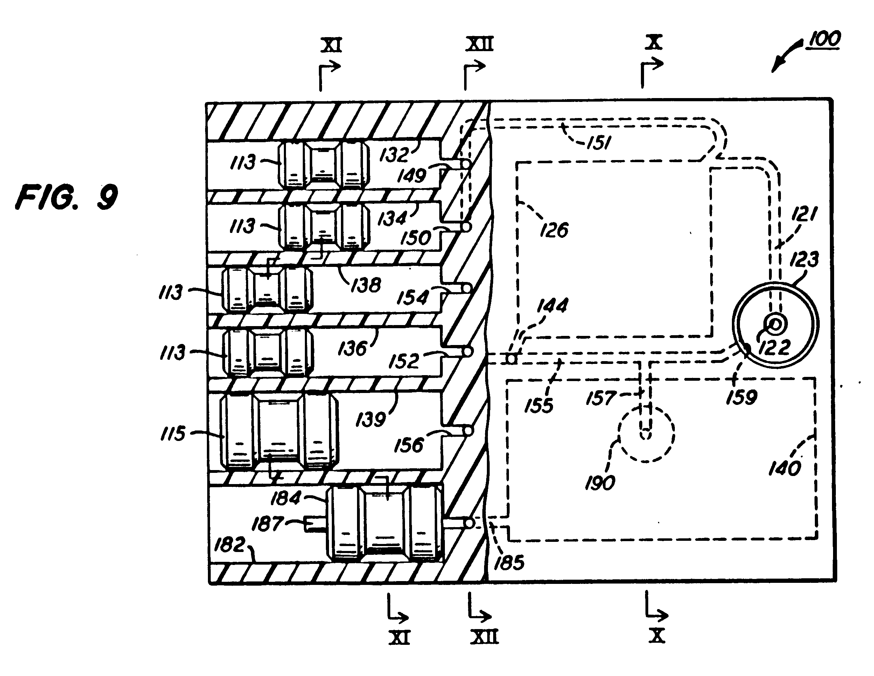

It is not essential that all compartments 30 to 38 be sealed off from the atmosphere during and after amplification, as shown in the embodiment of Figures 7 and 8, provided they are constructed to prevent a backwash of amplified DNA from entering them. Parts similar to those previously described bear the same reference numeral, to which the distinguishing suffix B has been appended.

Cuvette 10B has all the compartments 26B, 30B, 32B, 34B, 36B, 38B, 40B, and 42B as before, with their passageways interconnecting them so as to function as before. However, each and every one has a liquid injection aperture 70 at the periphery 16B providing, with connecting passageway 72, a fluid path from the atmosphere to the respective compartment. In such a construction, only compartment 26B has liquid in it at the time of DNA amplification, namely sample DNA and the amplifying reagents. (Its injection aperture 22B is closed at this time.) The other compartments can be left open, because of the temporary heat seals formed at their junction with their exit passageways. As an additional safeguard, a check valve 80 can be inserted into passageway 54B to prevent a backwash of DNA into those compartments. Such a valve is conventional, and can comprise, for example, a ball valve comprising a seat 82 and a ball 84 as shown in Figure 8. The ball 84, when pushed back upstream, seats on seat 82 to stop flow. Ball 84 is free, however, to flow downstream and its amount of travel is limited by a small stop 86.

Valve 80 is preferably located in trunk passageway 54B, as shown in Figure 7, since this allows one valve to serve all the storage compartments, and it is out of the way of path A, that is, it does not represent an obstacle to the passage of the pressure roller.

After each storage compartment receives its appropriate liquid from a pipette (Figure 7) and before the pressure roller moves down path A, each aperture 70 is closed tightly in a manner similar to the closure of aperture 22B. Alternatively, apertures 70 can be used as a fill technique to preincorporate all the reagents.

In the remaining embodiments, the means for moving the liquids containing, for example, amplified DNA and the detection material, is a piston in a piston chamber, instead of a collapsible flexible wall of the compartments. The piston chamber forms the appropriate compartment, and the pistons are the equivalent of the flexible walls of the compartments. The piston-piston chamber arrangement can also be used for fluidly interconnecting all the compartments.

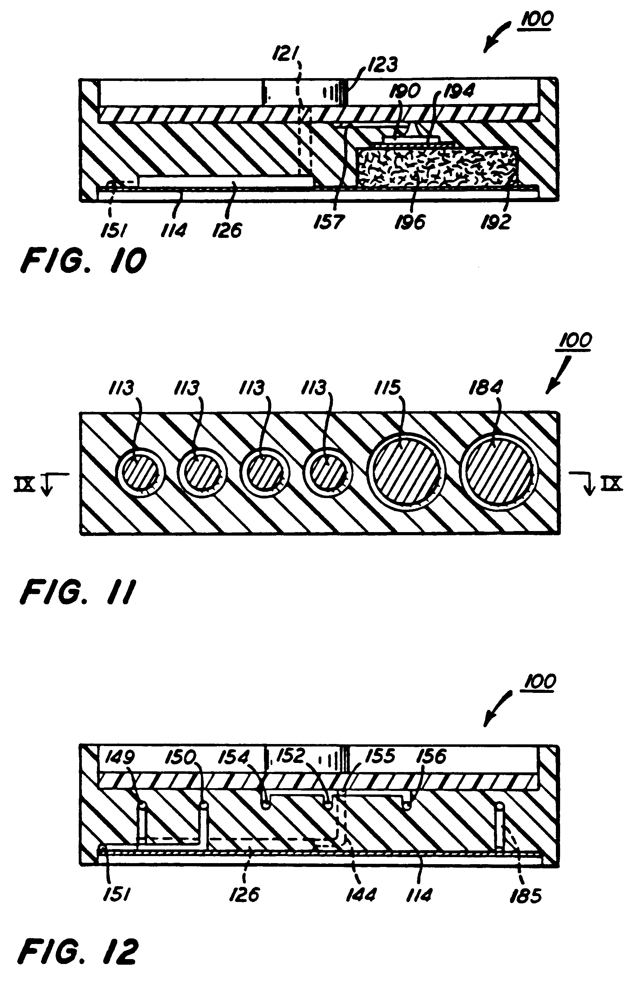

In Figures 9 to 12, a cuvette 100 comprises a reaction compartment 126 having a thermal transfer wall 114, as shown in Figures 10 and 12, a detection material storage compartment 132, a wash storage compartment 134, a leuco dye storage compartment 136, a stop solution storage compartment 138, and another wash storage compartment 139. Each compartment 126, 132, 134, 136, 138, 139 has an associated passageway 144, 149, 150, 152, 154, 156 connected to it. Thermal transfer wall 114 is preferably constructed as described previously. Each storage compartment 132, 134, 136, 138, 139 functions as a piston chamber. Mounted within each chamber is a piston 113, 115, which is disposed outside of the reagent in the compartment, that is, along an edge of the cuvette 100.

Preferably, the pistons are double-sealing as shown, and include means such as a slot (not shown) for positively engaging a driver actuator for that compartment (not shown).

Passageways 149 and 150 are connected together to form passageway 151, as shown in Figures 9 and 12. This passageway 151 connects with reaction compartment 126. A DNA sample is fed to the reaction compartment 126 via passageway 121 from liquid ingress aperture 122. This aperture is provided with an exterior shoulder 123, see Figure 9.

Passageways 152, 154 and 156 join together, as shown in Figure 12, to form passageway 155 into which passageway 144 feeds from compartment 126, see Figure 9. Passageway 155 then branches to form passageway 157 which leads to compartment 140, and a vent passageway which exits at 159 within shoulder 123. Shoulder 123 is internally threaded (not shown) to receive an externally threaded stopper (also not shown). The ingress aperture 122 and vent aperture 159 can both be sealed off by the stopper.

Since there is no flexible wall for expansion, a separate piston 184 and piston chamber 182 are provided to allow air expansion from detection compartment 140. This piston-piston chamber arrangement 184, 182 is shown in Figures 9, 11 and 12. Chamber 182 is connected via passageway 185 to the bottom of compartment 140. This can be done by manually withdrawing piston 184 within its chamber. A stem 187 can project from piston 184 for assisting in withdrawal of that piston.

Referring in particular to Figure 9, flow-through detection compartment 140 has an upper portion 190, into which liquid first enters as it is transferred from other compartments, and a lower portion 192, as shown more clearly in Figure 10, separated from the upper portion by a permeable membrane 194. Lower portion 192 is substantially filled by an absorbent member 196, which is intended to absorb all the excess liquid which enters compartment 140. Membrane 194 is preferably a cast, woven or electrooptically machined, microfiltration membrane. Any suitable material can be used for absorbent member 196, for example, cellulose acetate.

Membrane 194 functions to aid in separation of free, unreacted detection label, from those hybridized to the DNA. The detection probes in compartment 132 are designed both to hydridize onto amplified DNA in compartment 126, and to attach to membrane 194 (or to beads which are trapped by the membrane) once the liquid reaches compartment 140. Such probes also include a label such as horseradish peroxidase, which reacts with the leuco dye and peroxide when those materials reach compartment 140.

Filling of compartments 132, 134, 136 and 138 can be achieved by adding liquid and then inserting the pistons. Alternatively, prepackaged ampules (not shown) can be inserted, followed by the pistons, the ampules being frangible so that as pressure is applied by the piston, the ampule breaks open to release the liquid.

Transfer of liquid in cuvette 100 is controlled by the pistons 113, 115, and 184, piston 184 being used to create the vacuum which allows the other pistons to advance.

Alternatively (not shown), an additional compartment and associated piston can be included to feed additional enzyme into compartment 126, so that any deactivation of enzyme by the denaturing step can be countered by the addition of more enzyme.

Use of cuvette 100 is as follows:-

The pistons 113, 115, 184 start in the positions as shown in Figure 9. Sample DNA is introduced via a pipette at aperture 122, and passes through passageway 121 into compartment 126. In compartment 126, the amplification reagents may already be present, or they may be co-introduced with the DNA. The vent aperture 159 and passageway 155 allow the air in compartment 126 to be pushed out by the advancing liquid. A stopper is then inserted into shoulder 123, sealing off apertures 122 and 159. Thermal cycling is carried out on compartment 126, using thermal transfer wall 114, until the desired DNA amplification is achieved. Up until this point, the pistons have not been moved.

Next, piston 113 of compartment 132 is advanced to push the detection material stored in compartment 132 into compartment 126. In the case of the avidin-bead capture method, the contents of compartment 126 preferably include polymer beads to which is bound a biotinylated primer capable of extending with the amplified DNA in an annealing step, the primer being bound via avidin, to copy the amplified DNA. The compartment 126 also includes a detection probe, that is, a nucleotide constructed to hybridize with the extended primer attached to a moiety such as horseradish peroxidase. Some wash solution from compartment 134 can be pushed in by piston 113 to insure all of the detection reagents are present in compartment 126, if desired. Mixing can then be achieved by agitating the entire cuvette by any conventional means. The detection probes are then hydridized to the amplified DNA in compartment 126 by applying thermal control in conventional steps, through thermal transfer wall 114, for example, by heating at 42°C for five minutes.

Next, additional wash solution is pushed in from compartment 134, to wash the hybridized liquid from compartment 126 to detection compartment 140. Piston 115 is also advanced to push wash from compartmet 139 through passageway 156 and 155 to wash any hybridized liquid still remaining in passageway 155, into compartment 140, thus isolating any remaining hybridized liquid in passageway 144 from the leuco dye which is to follow. Enough wash solution is passed through compartments 126 and 140 to insure that all material, such as free detection probes not bound to the trapping means, passes through membrane 194 and into absorbent member 196. It is for this wash step, primarily, that piston 184 needs to be withdrawn to prevent back pressure resisting the transfer of liquid from compartment 126 to compartment 140.

Next, first the leuco dye of compartment 136 and then the stop solution of compartment 138 are advanced into passageways 155 and 157 and then into compartment 140. This is achieved by advancing their respective pistons 113. This will cause appropriate dye formation at membrane 194 if the amplified DNA is present. (If it is not present, since all free detection material has already washed through into absorbent member 196, no color will form and the test will indicate "negative".)

It is not essential that a separate detection compartment be present in order to have a detection site. As is explained regarding the next embodiment, the detection site can be in the reaction compartment itself. Parts similar to these previously described bear the same reference numeral, to which the distinguishing suffix "C" is appended.

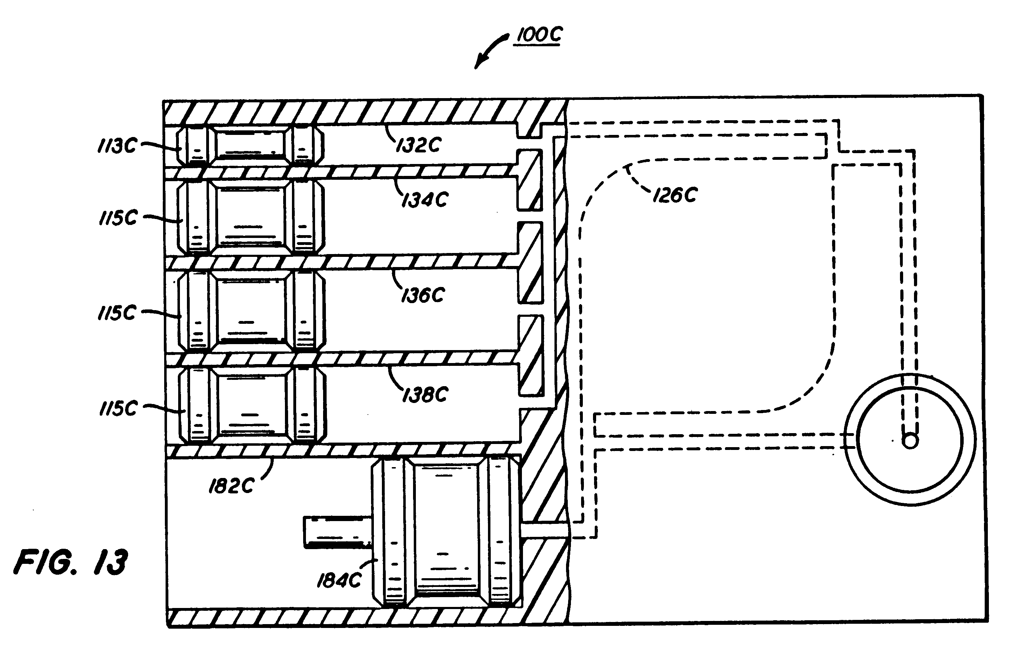

In Figure 13, cuvette 100C has compartments 126C, 132C, 134C, 136C, 138C and their passageways leading to and from each other and from ingress aperture 122C as before. Pistons 113C, 115C and 184C are used to transfer liquid as before, after the DNA amplification as described earlier. However, in this embodiment, compartment 132C includes as detection reagents, magnetic beads formed from polymers containing magnetic fillers, to which have been bonded the hydridizing material with matching DNA sequences. This is intended to hybridize to one end, for example, of the amplified DNA. The other end of that amplified DNA is intended to hybridize to a detection probe bearing the horseradish peroxidase, as described above.

In this embodiment, separation of the free detection probes not yet hybridized to DNA, from those which are, is achieved as follows:-

When wash solution is injected from compartment 134C, a magnetic field is supplied below compartment 126C, to retain the bead reagents and any detection probe hybridized to an amplified DNA. This causes free detection probes and their labels to be washed out of compartment 126C and into chamber 182C, in which piston 184C has been withdrawn to make room. The magnetic field is further maintained while the leuco dye and the stop liquids are transferred into the reaction compartment 126C, causing color to form there if any amplified DNA is present.

Another alternative to the cuvette described with reference to Figures 8 to 12 is to extend the compartments 132, 134, 136, 138, 182, or their "C" counterparts (as described with reference to Figure 13), so that they project 90° out of the plane of Figure 8 (not shown) giving an L-shape to the cuvette. Such an arrangement has the advantages of simplifying mold design and fabrication.

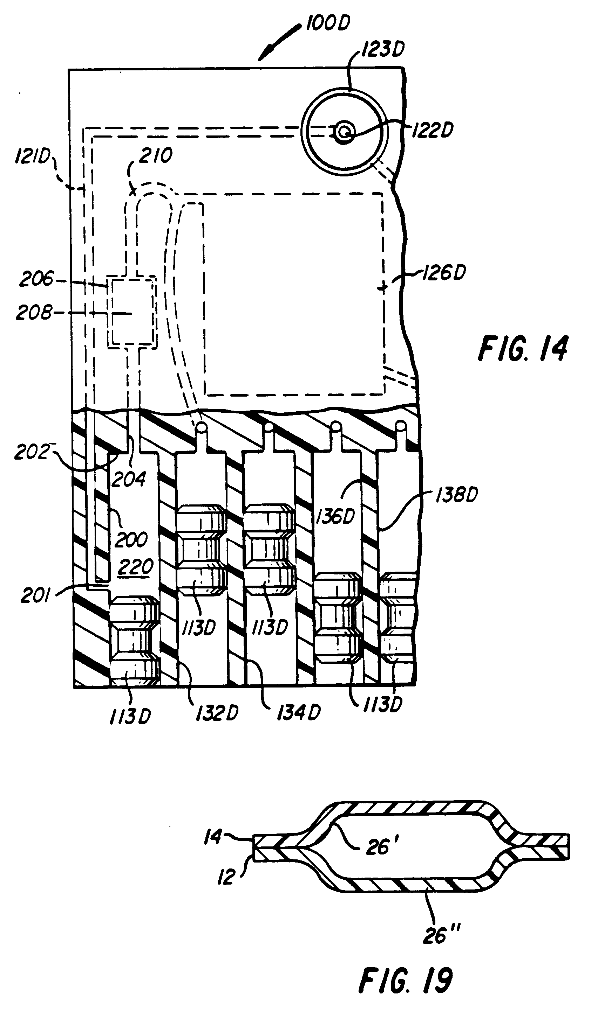

It is possible to extract DNA from cells within the cuvette of the invention, instead of at a stage prior to the use of the cuvette. In such a case, the cuvette is preferably constructed as shown in Figure 14. Parts similar to those previously described bear the same reference numeral, to which the distinguishing suffix "D" is applied.

Cuvette 100D has the same compartments 126D, 132D, 134D, 136D and 138D as before, with pistons 113D being used in the storage compartments. Shoulder 123D protects liquid ingress aperture 122D, and detection is done at a membrane (not shown), all as discussed before. However, passageway 121D, instead of delivering the pipetted liquid sample direct to the reaction compartment 126D, it delivers it to an extraction compartment 200. The liquid sample in this case is whole blood or a solution of blood cells, from which the DNA is to be extracted. At the time this liquid sample is added to the cuvette, extracting agents discussed below can be optionally added. Alternatively, they can be pre-incorporated into compartment 200.

A piston 113D is used in compartment 200, as with the other similar compartments, except that it is fully withdrawn, as shown, to provide maximum room for the introduced sample. Passageway 121D enters compartment 200 at a point 201 just below piston 113D.

At the opposite end 202 of compartment 200, a passageway 204 fluidly connects to an intermediate compartment 206, in which is disposed a filter 208 which the liquid must traverse, in order to reach compartment 126D. Filter 208 has pore sizes adequate to retain cellular fragments in the filter and to pass extracted DNA. For example, a filter made of nylon or polypropylene with pore sizes of about 0.45 $\mu$m is particularly useful.

From compartment 206, a passageway 210 carries extracted DNA and solvent (e.g., water) into compartment 126D.

In use, the liquid sample is injected into compartment 200, preferably along with extraction agents, if any. Any DNA extraction protocol can be used, along with concommitant extraction agents, such as surfactants. Highly preferred is a simple heating of the solution to a temperature of about 95°C for about 5min. Such heating is effective to denature the proteins and lyse the cells. As aids in this extraction method, dextran can be optionally added as a 3wt% solution, along with a 10wt% solution of TX-100 (Registered TM), which is a non-ionic surfactant available from Rohm and Haas. The heating of compartment 200 can be more rapidly achieved by constructing at least a portion 220 of the wall of the compartment from aluminium. The aluminium extends to the bottom exterior of the cuvette (not shown as a separate surface), and the application of heat to this region of the cuvette adjacent compartment 200 is thus effective to heat compartment 200.

After a suitable incubation period, the contents of compartment 200 are pushed to filter 208 by advancing piston 113D.

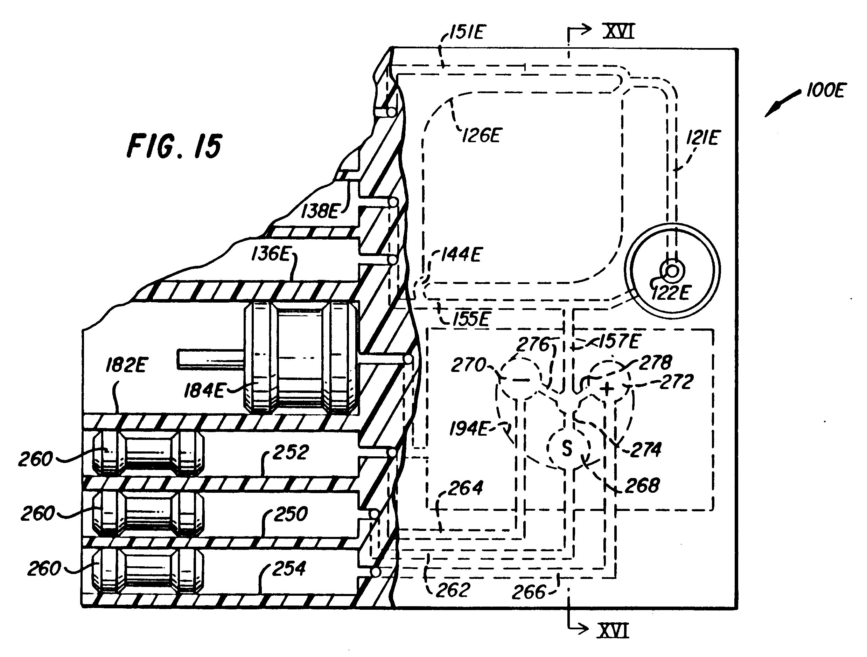

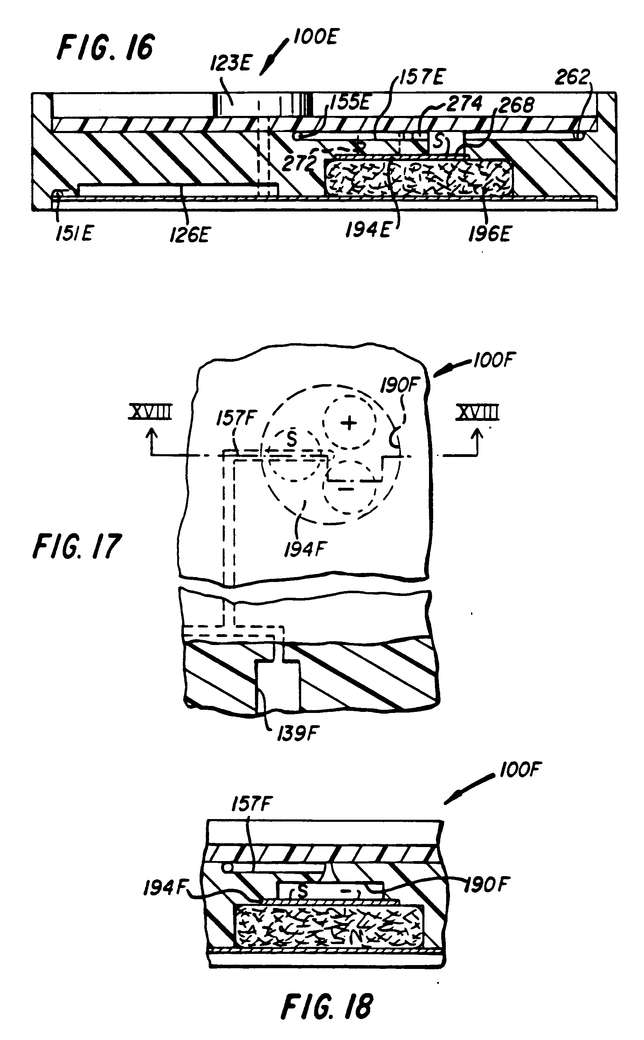

In some instances, it may be desirable to have a positive and a negative control in the detection compartment, along with the detection site for the DNA of choice. Figures 15 and 16 are illustrative of a modification which provides this. Parts similar to those previously described bear the same reference numeral, to which the distinguishing suffix "E" is applied.

In Figure 15, a cuvette 100E has compartments 126E, 136E, 138E and 182E as before, with appropriate pistons 184E, etc. A passageway 121E carries liquid sample from aperture 122E to reaction compartment 126E, and passageway 151E also feeds liquid to that compartment. Biotinylated primers are delivered from a suitable location, and leuco dye and stop solution are delivered via passageways 155E and 157E to the detection site. Passageway 144E provides access of the DNA product produced in compartment 126E, to those passageways 155E and 157E. Liquid coming from passageway 157E encounters a detection membrane 194E disposed in contact with absorbent member 196E, as shown in Figure 16, This is as described with reference to the previous embodiments.

However, unlike previous embodiments, there are separate regions at membrane 194E for the sample DNA detection, labeled "S"; for a positive control, labeled "+"; and for a negative control, labeled "-", as shown in Figure 15. The purpose of the positive control is to ensure that the reagents will produce a signal for DNA (preferably a color), if the DNA is present. This alerts the user if the reagents are defective in any way by failing to produce a signal at the "+" region. The negative control on the other hand should not produce a signal. The primary purpose of the negative control is to give the user a background color against which the sample color is to be compared. For example, a faint background color may occur in the reagents for extraneous reasons, and it is important that the sample color be significantly greater in density than this, before a "positive" read is attributed to the test.

There are several ways in which this can be done. In the embodiment of Figures 15 and 16, the method used is the avidin-bead capture. This method features the use of avidin or streptavidin covalently attached to beads, biotinylated primers, and labeled detection probes as part of the detection material. Preferably at least the probes are kept in storage compartments 250, 252 and 254. Each compartment is dedicated to a single type of detection probe. Compartment 250 has the sample DNA probes, 252 the negative control probes, and 254 the positive control probes. Pistons 260 are used to pressurize their respective compartments, preferably simultaneously, to force the contents out through respective passageways 262, 264 and 266 into the detection compartment 268, 270 and 272 associated with each probe and each probe passageway. In this way, each detection compartment has only its probe in it, and none of the other two.

To feed amplified DNA and other reagents to all three detection compartments, passageway 157E preferably splits into three branches 274, 276, and 278 which connect with the respective detection compartments 268, 270, 272.

It will be readily appreciated that two of the three probes have a genetic material which is complementary to the appropriate DNA. The probe for the sample has a genetic complement to the targeted DNA of the sample. The positive probe has a genetic complement for a DNA material which is always present, which at least in the case of blood cells, is preferably beta-globin. The negative probe has a genetic complement which matches no known amplified DNA from the sample. This is done most easily by constructing the probe complement with any genetic code which is random in sequence, hence a "nonsense" code.

As will be apparent, the process works as follows:-

The avidin-bearing beads are preferably stored with the probes in compartments 250, 252 and 254. As amplified DNA is supplied via passageways 274, 276 and 278 to the respective detection compartments, pistons 260 are advanced to push the appropriate probes and beads into those detection compartments. Those compartments are appropriately heated to cause the amplified DNA to hybridize specifically in compartments 268 and 272 to the appropriate probe from compartments 250 and 254. Preferably, no hybridization occurs in detection compartment 270, since there should be no "nonsense" DNA present to react with the negative control probe. Wash solution is then pushed through the detection compartments to wash any labeled probes not hybridized and thus not attached to beads into the absorbent member 196E through membrane 194E. The wash is followed by contact with the leuco dye solution, and then contact with the stop solution.

Another method which can be used is the so-called oligo capture technique. In this technique, as shown in Figures 17 and 18, the labeled probes are stored in immobilized form, already on the detection membrane prior to the introduction of amplified DNA. Parts similar to those previously described bear the same reference numeral, to which the distinguishing suffix "F" is appended.

The oligo capture method used in this embodiment features probes immobilized either on the membrane, or on the above-mentioned beads which are trapped on or in the membrane, such probes containing genetic material which is complementary to the appropriate DNA. In another compartment (139F), labeled avidin for reaction with biotin on the amplified DNA is stored for which the label can be, for example, horseradish peroxidase.

In Figures 17 and 18, cuvette 100F is identical to that of the embodiment of Figure 9, namely cuvette 100 except for the detection compartment 190F and what is stored in storage-compartment 139F. More specifically, the sample DNA probe is immobilized on portion "S" of membrane 194F, the positive control probe is immobilized on portion " + ", and the negative control probe is immobilized on portion "-". Each portion of membrane 194F bearing such probes is preferably not in contact with the other portions.

The procedure in this case is to force amplified DNA via passageway 157F into detection compartment 190F, to flow over the entire surface of membrane 194F. Appropriate heating causes amplified target DNA to hybridize specifically and thus attach to the probe in the "S" area, the ubiquitous DNA to attach to the probe in the " + " area, and preferably nothing to hybridize at the "-" area. A wash solution is forced into compartment 190F, e.g. from 139F or elsewhere, followed by avidin-label, which then reacts with the biotinylated product now hybridized to either the amplified target DNA or the positive control DNA. A wash solution is then added, followed by leuco dye and stop solution.

## Claims

1. A disposable cuvette (10; 10A; 10B; 100; 100C; 100D; 100E; 100F) for carrying out amplification and detection of nucleic acid material comprising:

   a first compartment (40; 40A; 40B; 126C);

   a plurality of further compartments (26, 30, 32, 34, 36, 38, 42; 61, 26A, 30A, 38A; 26B, 30B, 32B, 34B, 36B, 38B, 42B; 126, 132, 134, 136, 138, 139, 140, 182; 132C, 134C, 136C, 138C; 126D, 132D, 134D, 138D; 126E, 136E, 138E, 182E, 250, 252, 254), at least one of the further compartments containing material to amplify and detect nucleic acid material;

   fluid connection means (44, 48, 49, 50, 52, 54; 62, 44A, 48A, 49A, 50A, 52A, 54A; 54B; 144, 149, 150, 151, 152, 154, 155, 156, 157, 185; 144E, 151E, 157E, 262, 264, 266; 157F) for connecting at least one of the further compartments with the first compartment (40; 40A; 40B; 126C) so that the material retained in the at least one further compartment is transferable to the first compartment (40; 40A; 40B; 126C) when pressure is applied to the material retained in that compartment;

   a detection site (39; 190; 190F) for immobilizing the nucleic acid material for detection after amplification; and

   thermal transfer means for allowing active or passive cycling of material in the cuvette; characterized in that the first compartment (40; 40A; 40B; 126C) and the detection site (39; 190; 190F) are closed to fluid flow to locations outside of the cuvette so that aerosols are not emitted during amplification and detection.

2. A cuvette according to claim 1, wherein the thermal transfer means comprises a wall of the first compartment (40; 40A; 40B; 126C) which is made from a heat transferable material.

3. A cuvette according to claim 2, wherein the heat transfer wall has a thermal path length of no more than about 0.3mm and a thermal resistance for a cross-sectional area of 1cm$^2$ of no more than about 5.0°C/W.

4. A cuvette according to any one of the preceding claims, wherein at least one wall of the at least one further compartment is sufficiently flexible as to allow external pressure to compress the compartment and to effect transfer of material therein to the first compartment (40;

40A; 40B; 126C).

5. A cuvette according to claim 4, wherein the further compartments (26, 30, 32, 34, 36, 38, 42; 61, 26A, 30A, 38A; 26B, 30B, 32B, 34B, 36B, 38B, 42B) and their fluid connection means (44, 48, 49, 50, 52, 54; 62, 44A, 48A, 49A, 50A, 52A, 54A; 54B) are disposed so that material retained in each compartment can be forced out in a prescribed sequence by applying external pressure linearly along the outside surfaces of the cuvette.

6. A cuvette according to claim 4 or 5, wherein external pressure is applied by roller means (60) which advances over an external surface of the cuvette.

7. A cuvette according to any one of claims 4 to 6, wherein one of the further compartments (61) contains water and is fluidly connected to all of the other further compartments (26A, 30A, 38A), this one compartment being positioned so as to be the first to be pressurized.

8. A cuvette according to any one of claims 1 to 3, wherein one of the further compartments is a reaction compartment (26; 26A; 26B; 126; 126C; 126D; 126E) into which the nucleic acid material is introduced.

9. A cuvette according to claim 8, wherein the reaction compartment (26; 26A; 26B; 126; 126C; 126D; 126E) has unreacted amplifying agents (28) pre-incorporated therein.

10. A cuvette according to claim 9, wherein the unreacted amplifying agents are polymerase enzyme, primer nucleic acids and nucleotides.

11. A cuvette according to claim 9, wherein the unreacted amplifying agents are TAQ polymerase, primer nucleic acids and nucleotides.

12. A cuvette according to any one of claims 1 to 7, further including pressurizing means for pressurizing the material retained in the at least one further compartment to effect transfer of material to the first compartment.

13. A cuvette according to claim 12, wherein the pressurizing means includes a first piston-cylinder arrangement (113, 115) housed within the at least one further compartment.

14. A cuvette according to claim 12 or 13, wherein the pressurizing means includes a second piston-cylinder arrangement (182, 184; 182C, 184C; 182E, 184E) which is fluidly connected to the first compartment (140; 126C) so that when the second piston (184; 184C; 184E) is withdrawn in its cylinder, it relieves pressure in the first compartment.

15. A cuvette according to any one of claims 12 to 14, wherein one of the further compartments is a reaction compartment (126D) into which the nucleic acid material is introduced, the cuvette further including an extraction compartment (200) positioned fluidly upstream of the reaction compartment (126D), and a filter (208) interposed in the fluid path from the extraction compartment (200) to the reaction compartment (126D), the extraction compartment (200) enabling DNA to be separated from at least blood cells, cellular fragments being separated from the DNA by the filter (208) so that only the DNA proceeds to the reaction compartment (126D).

16. A cuvette according to any one of the preceding claims, wherein the detection site (39; 190; 190F) includes a probe containing genetic material which is complementary to a DNA material, and therefore hydridizes with that DNA material when appropriately heated.

17. A cuvette according to claim 16, wherein the detection site further includes a positive control probe containing genetic material which is complementary to an amplified DNA always present in a blood sample.

18. A cuvette according to claim 17, wherein the positive control probe genetic material is complementary to beta-globin.

19. A cuvette according to claim 17 or 18, wherein the positive control probe is immobilized on a portion of the detection site, in the absence of amplified DNA.

20. A cuvette according to any one of claims 16 to 19, wherein the detection site further includes a negative control probe containing genetic material which is not complementary to any DNA expected to be present after amplification of a blood sample.

21. A cuvette according to claim 20, wherein the negative control probe is immobilized on a portion of the detection site, in the absence of amplified DNA.

22. A cuvette according to claim 20 or 21, wherein the positive control and the negative control probes are immobilized on a portion of the detection site in the absence of any amplified DNA, each of the immobilized site portions being substantially free of the other control probe.

23. A cuvette according to any one of the preceding claims, wherein the detection site (39) comprises a supporting sheet (41) on which are disposed a plurality of piles (43, 43′, 43″, 43‴), each pile comprising a polymer bead to which is attached a DNA probe.

24. A cuvette according to any one of claims 1 to 22, wherein the detection site (39) comprises a supporting sheet (41) on which are disposed a plurality of piles (43, 43′, 43″, 43‴), each pile comprising a polymer bead to which is attached avidin.

25. A cuvette according to any one of the claims 16 to 22, wherein the detection site is in the first compartment (126C), and material for detecting the nucleic acid material includes a bead comprising magnetizable material.

26. Apparatus for amplifying and detecting DNA including a cuvette according to any one of the preceding claims.

27. Apparatus for amplifying and detecting DNA, comprising

a cuvette containing i) a plurality of compartments and means for interconnecting each of them to at least one other compartment, the compartments including a) at least one reaction compartment for amplifying DNA strands, b) at least one detection compartment for detecting amplified DNA and including a detection site, and c) means for delivering a detection material to amplified DNA strands;

ii) means for permitting active or passive cycling of the contents of the reaction compartment through a temperature range of from about 30°C to about 95°C; and

iii) liquid access means connected only to the at least one reaction compartment for allowing the injection into the reaction compartment of a sample DNA for amplifying;

characterized in that the cuvette further includes

iv) means for sealing the cuvette against passage of DNA after sample DNA is injected;

and the apparatus further includes means for moving at least the detection material and a DNA strand into the detection compartment and onto the detection site;

so that once a DNA sample is injected into the compartments and the access aperture is closed, the fluid contents of the compartments are contained against contact by the operator and environment during the entire amplification and detection reaction.

28. A method for amplifying and detecting nucleic acid material in a closed cuvette comprising the steps of

a) injecting a sample of nucleic acid material into a cuvette which comprises a plurality of compartments including a reaction compartment wherein amplifying reagents are present, and a storage compartment for use with a detection material, at least one of the compartments including a detection site, and means for interconnecting the compartments to provide fluid transfer;

b) closing off permanently the portions of the cuvette containing the nucleic acid material to lock all nucleic acid into the cuvette;

c) amplifying the nucleic acid material by cycling the cuvette through temperature changes preselected to cause the reagents to be effective;

d) fluidly transferring amplified nucleic acid material from the reaction compartment to the detection site;

e) fluidly transferring detection material to the detection site; and

f) detecting the amplified nucleic acid material at the detection site with the detection material, the nucleic acid material remaining confined within the cuvette.

29. A method according to claim 28, wherein the step c) includes the further step of transferring heat across a thermal transfer wall of the reaction compartment, both into and out of the compartment, the thermal transfer wall comprising at least one thermally conductive material.

30. A method according to claim 29, wherein the thermal transfer wall has a thermal path length of no more than about 0.3 mm and a thermal resistance for a cross-sectional area of $1cm^2$ of no more than about 5.0°C/watt.

31. A method according to any one of claims 28 to 30, wherein the cuvette further includes a first piston-cylinder arrangement in one of the compartments which is fluidly connected with the reaction compartment so that the advance of the piston in the compartment causes pressure to be increased in the reaction compartment, and a second piston-cylinder arrangement in another of the compartments which is fluidly connected to the detection site so that when the second piston is withdrawn in its cylinder, it relieves pressure at the detection site and the step d) comprises the step of advancing the first piston while withdrawing the second piston.

32. A method according to any one of claims 28 to 30, wherein at least one wall of the compartments is sufficiently flexible as to allow external pressure to compress the compartments to force liquid transfer out of the compartments, and wherein the step d) comprises the step of applying exterior pressure to the flexible walls of the compartments in a prescribed sequence.

33. A method according to any one of claims 28 to 30, wherein the detection reagents include a bead comprising a magnetizable material and wherein the steps d) - f) comprise transfer of the beads to the reaction compartment, attaching the beads to the amplified nucleic acid material, attaching the detection material to the amplified material, and washing away unattached detection material in the present of a magnetic field that retains the beads and attached detection material within the reaction compartment.

34. A method according to any one of claims 28 to 30, wherein the steps d) and e) occur sequentially by pressurizing first the reaction compartment and thereafter a storage compartment.

35. A method according to any one of claims 28 to 30, wherein the steps d) and e) occur by pressurizing the storage compartment and the reaction compartment simultaneously, and retarding the flow of detection material until amplified nucleic acid material has been transferred.

36. A method according to any one of claims 28 to 30, and further including as a step prior to the step e), the step of reconstituting detection material deposited in dried form in a storage compartment, by transferring pre-incorporated water to the dried material from a storage compartment.

37. A method according to any one of claims 28 to 31, wherein step a) comprises the step of injecting at least blood cells and optional DNA extraction agents into a predetermined one of the compartments to form a solution; and before step c), further including the steps of:

i) extracting DNA from the cells in the predetermined compartment; and
ii) after a suitable incubation period, forcing the solution of extracted DNA and cell fragments through a filter disposed between the predetermined one compartment and the reaction compartment, the filter being sized to retain cellular fragments and to pass DNA.

**Patentansprüche**

1. Einwegküvette (10;10A; 10B; 100; 100C; 100D, 100E; 100F) für die Amplifikation und Detektion von Nukleinsäurematerial mit
einer ersten Kammer (40; 40A; 40B; 126C),
einer Vielzahl weiterer Kammern (26, 30, 32, 34, 36, 38, 42; 61, 26A; 30A; 38A; 26B; 30B, 32B, 34B, 36B, 38B, 42B; 126, 132, 134, 136, 138, 139, 140, 182; 132C, 134C, 136C, 138C; 126D, 132D, 134D, 138D; 126E, 136E, 138E, 182E, 250, 252, 254), von denen mindestens eine Kammer ein Material für die Amplifikation und Detektion von Nukleinsäurematerial enthält,
Strömungsverbindungsmitteln (44, 48, 49, 50, 52, 54; 62, 44A, 48A, 49A, 50A, 52A, 54A; 54B; 144, 149, 150, 151, 152, 154, 155, 156, 157, 185; 144E, 151E, 157E, 262, 264, 266; 157F), die mindestens eine der weiteren Kammern derart mit der ersten Kammer (40; 40A; 40B; 126C) verbinden, daß das in mindestens der einen weiteren Kammer enthaltene Material in die erste Kammer (40; 40A; 40B; 126C) übertragbar ist, wenn das in dieser Kammer enthaltene Material mit Druck beaufschlagt wird,
einer Detektionsstelle (39, 190; 190F), an der das Nukleinsäurematerial für die Detektion nach der Amplifikation immobilisierbar ist, und thermischen Transfermitteln, die eine aktive oder passive Cyclusbehandlung des Materials in der Küvette ermöglichen,
**dadurch gekennzeichnet,** daß die erste Kammer (40; 40A; 40B; 126C) und die Detektionsstelle (39; 190; 190F) so verschlossen sind, daß keine Flüssigkeit zu außerhalb der Küvette liegenden Stellen strömt und während der Amplifikation und Detektion keine Aerosole abgebbar sind.

2. Küvette nach Anspruch 1, dadurch gekennzeichnet, daß die thermischen Transfermittel eine aus einem Wärme übertragenden Material hergestellte Wandung der ersten Kammer (40; 40A; 40B; 126C) umfassen.

3. Küvette nach Anspruch 2, dadurch gekennzeichnet, daß die Wärme übertragende Wandung eine Wärmeübertragungsstrecke von höchstens etwa 0,3 mm bildet und für einen Querschnittsbereich von 1 cm² einen thermischen Widerstand von höchstens etwa 5,0 °C/W besitzt.

4. Küvette nach einem der vorhergehenden Ansprüche, dadurch gekennzeichnet, daß mindestens eine Wandung der mindestens einen weiteren Kammer so flexibel ist, daß die Kammer unter der Wirkung eines von außen auf sie ausgeübten Drucks zusammenpreßbar und das in dieser Kammer enthaltene Material zu der ersten Kammer (40; 40A; 40B; 126C) übertragbar ist.

5. Küvette nach Anspruch 4, dadurch gekennzeichnet, daß die weiteren Kammern (26, 30, 32, 34, 36, 38, 42; 61, 26A, 30A, 38A; 26B, 30B, 32B, 34B, 36B, 38B, 42B) und ihre Strömungsverbindungsmittel (44, 48, 49, 50, 52, 54; 62, 44A, 48A, 49A, 50A, 52A, 54A; 54B) so angeordnet sind, daß das in jeder Kammer enthaltene Material in einer vorbestimmten Reihenfolge aus der Kammer herausdrückbar ist, indem die Küvette von außen linear an ihren Außenflächen Druck ausgesetzt wird.

6. Küvette nach Anspruch 4 oder 5, dadurch gekennzeichnet, daß der Druck mittels einer sich über eine Außenfläche der Küvette bewegenden Rolle (60) ausübbar ist.

7. Küvette nach einem der Ansprüche 4 bis 6, dadurch gekennzeichnet, daß eine der weiteren Kammern (61) Wasser enthält und mit allen anderen weiteren Kammern (26A, 30A, 38A) in Strömungsverbindung steht und daß diese eine Kammer so angeordnet ist, daß sie als erste dem Druck aussetzbar ist.

8. Küvette nach einem der Ansprüche 1 bis 3, dadurch gekennzeichnet, daß eine der weiteren Kammern eine Reaktionskammer (26; 26A; 26B; 126; 126C; 126D; 126E) darstellt, in die das Nukleinsäurematerial einführbar ist.

9. Küvette nach Anspruch 8, dadurch gekennzeichnet, daß in die Reaktionskammer (26; 26A; 26B; 126; 126C; 126D; 126E) noch nicht umgesetzte Amplifikationsmittel (28) vorab inkorporiert sind.

10. Küvette nach Anspruch 9, dadurch gekennzeichnet, daß die noch nicht umgesetzten Amplifikationsmittel aus Polymerase-Enzym, Starter-Nukleinsäuren und Nukleotiden bestehen.

11. Küvette nach Anspruch 9, dadurch gekennzeichnet, daß die noch nicht umgesetzten Amplifikationsmittel aus TAQ-Polymerase, Starter-Nukleinsäuren und Nukleotiden bestehen.

12. Küvette nach einem der Ansprüche 1 bis 7, dadurch gekennzeichnet, daß eine Druckeinrichtung vorgesehen ist, die das in der mindestens einen weiteren Kammer enthaltene Material so mit Druck beaufschlagt, daß das Material in die erste Kammer übertragbar ist.

13. Küvette nach Anspruch 12, dadurch gekennzeichnet, daß die Druckeinrichtung eine erste Kolben-Zylinder-Anordnung (113, 115) enthält, die sich in der mindestens einen weiteren Kammer befindet.

14. Küvette nach Anspruch 12 oder 13, dadurch gekennzeichnet, daß die Druckeinrichtung eine zweite Kolben-Zylinder-Anordnung (182, 184; 182C, 184C; 182E, 184E) enthält, die mit der ersten Kammer (140; 126C) derart in Strömungsverbindung steht, daß beim Rückhub des zweiten Kolbens (184; 184C; 184E) in seinem Zylinder eine Druckentlastung in der ersten Kammer erfolgt.

15. Küvette nach einem der Ansprüche 12 bis 14, dadurch gekennzeichnet, daß eine der weiteren Kammern eine Reaktionskammer (126D) ist, in die das Nukleinsäurematerial einführbar ist, daß die Küvette eine Extraktionskammer (200) enthält, die strömungsmäßig vor der Reaktionskammer (126D) angeordnet ist, und daß ein Filter (208) in der von der Extraktionskammer (200) zur Reaktionskammer (126D) verlaufenden Strömungsbahn angeordnet ist, wobei in der Extraktionskammer (200) zumindest aus Blutzellen DNS abtrennbar ist und durch das Filter (208) Zellfragmente von der DNS abtrennbar sind, so daß nur die DNS in die Reaktionskammer (126D) gelangt.

16. Küvette nach einem der vorhergehenden Ansprüche, dadurch gekennzeichnet, daß die Detektionsstelle (39; 190; 190F) eine Sonde umfaßt, die einem DNS-Material komplementäres genetisches Material enthält und deshalb bei geeigneter Erhitzung mit dem DNS-Material hybridisiert.

17. Küvette nach Anspruch 16, dadurch gekennzeichnet, daß die Detektionsstelle außerdem eine Positivkontroll-Sonde umfaßt, die einer immer in einer Blutprobe vorhandenen amplifizierten DNS komplementär ist.

18. Küvette nach Anspruch 17, dadurch gekennzeichnet, daß das genetische Material der Positivkontroll-Sonde Beta-Globin komplementär ist.

19. Küvette nach Anspruch 17 oder 18, dadurch gekennzeichnet, daß die Positivkontroll-Sonde auf einem Abschnitt der Detektionsstelle in Abwesenheit von amplifizierter DNS immobilisiert ist.

20. Küvette nach einem der Ansprüche 16 bis 19, dadurch gekennzeichnet, daß die Detektionsstelle außerdem eine Negativkontroll-Sonde umfaßt, die genetisches Material enthält, das keiner DNS komplementär ist, deren Vorhandensein nach der Amplifikation einer Blutprobe erwartet wird.

21. Küvette nach Anspruch 20, dadurch gekennzeichnet, daß die Negativkontroll-Sonde auf einem Teil der Detektionsstelle in Abwesenheit von amplifizierter DNS immobilisiert ist.

22. Küvette nach Anspruch 20 oder 21, dadurch gekennzeichnet, daß die Positivkontroll-Sonde und die Negativkontroll-Sonde in Abwesenheit von amplifizierter DNS auf einem Teil der Detektionsstelle immobilisiert sind, wobei die immobilisierten Abschnitte jeweils im wesentlichen von der anderen Kontrollsonde frei sind.

23. Küvette nach einem der vorhergehenden Ansprüche, dadurch gekennzeichnet, daß die Dektionsstelle (39) ein Trägerblatt (41) aufweist, auf dem eine Vielzahl von Erhebungen (43, 43' 43'', 43''') angeordnet sind, von denen jede aus einem Polymerkügelchen besteht, an das eine DNS-Sonde gebunden ist.

24. Küvette nach einem der Ansprüche 1 bis 22, dadurch gekennzeichnet, daß die Detektionsstelle (39) ein Trägerblatt (41) umfaßt, auf dem eine Vielzahl von Erhebungen (43, 43', 43''; 43''') angeordnet ist, von denen jede aus einem Polymerkügelchen besteht, an das Avidin gebunden ist.

25. Küvette nach einem der Ansprüche 16 bis 22, dadurch gekennzeichnet, daß sich die Detektionsstelle in der ersten Kammer (126C) befindet und das Material zum Detektieren des Nukleinsäurematerials ein Kügelchen enthält, das ein magnetisierbares Material umfaßt.

26. Gerät zur DNS-Amplifikation und -Detektion mit einer Küvette nach einem der vorhergehenden Ansprüche.

27. Gerät zur DNS-Amplifikation und -Detektion mit einer Küvette, die
(i) eine Vielzahl von Kammern sowie Mittel zum Verbinden jeder dieser Kammern mit mindestens einer anderen Kammer enthält, wobei die Kammern a) mindestens eine Reaktionskammer zur Amplifikation von DNS-Strängen, (b) mindestens eine eine Detektionsstelle enthaltende Detektionskammer zur Detektion von amplifizierter DNS und c) Mittel zum Zuführen eines Detektionsmaterials zu amplifizierten DNS-Strängen umfassen,
(ii) Mittel aufweist, die eine aktive oder passive Cyclusbehandlung des Inhalts der Reaktionskammer in einem Temperaturbereich von etwa 30°C bis etwa 95°C erlauben, und
(iii) Flüssigkeitseinlaßmittel besitzt, die nur mit der mindestens einen Reaktionskammer verbunden sind und durch die eine DNS-Probe zur Amplifikation in die Reaktionskammer injizierbar ist,
dadurch gekennzeichnet, daß die Küvette außerdem
(iv) Verschlußmittel enthält, die verhindern, daß nach der Injektion der DNS-Probe DNS aus der Küvette herausgelangt, und
daß das Gerät Mittel aufweist, durch die mindestens das Detektionsmaterial und ein DNS-Strang in die Detektionskammer und auf die Detektionsstelle bewegbar sind, so daß
nach Injektion einer DNS-Probe in die Kammern und Schließen der Einlaßöffnung der flüssige Inhalt der Kammern während der gesamten Amplifikations- und Detektionsreaktion nicht mit der Bedienungsperson und der Umgebung in Berührung gelangt.

28. Verfahren zur Amplifikation und Detektion von Nukleinsäurematerial in einer geschlossenen Küvette, dadurch gekennzeichnet, daß in einer Reihe von Arbeitsschritten
a) eine Probe eines Nukleinsäurematerials in eine Küvette injiziert wird, die eine Vielzahl von Kammern enthält, einschließlich einer Reaktionskammer, in der Amplifikations-Reagenzien enthalten sind, und einer Speicherkammer, die in Verbindung mit einem Detektionsmaterial verwendbar ist, wobei mindestens eine der Kammern eine Detektionsstelle enthält und Mittel vorgesehen sind, die die Kammern zur Übertragung der Flüssigkeiten miteinander verbinden,
b) die das Nukleinsäurematerial enthaltenden Bereiche der Küvette dauerhaft verschlossen werden, so daß die gesamte Nukleinsäure in der Küvette eingeschlossen ist,

c) das Nukleinsäurematerial amplifiziert wird, indem die Küvette thermischen Cyclen unterworfen wird, die so vorgewählt werden, daß die Reagenzien ihre Wirkung entfalten,

d) das amplifizierte Nukleinsäurematerial in flüssiger Form aus der Reaktionskammer zu der Detektionstelle übertragen wird,

e) Detektionsmaterial in flüssiger Form zu der Detektionsstelle übertragen wird und

f) an der Detektionsstelle mittels des Detektionsmaterials das amplifizierte Nukleinsäurematerial nachgewiesen wird, wobei das Nukleinsäurematerial in der Küvette eingeschlossen bleibt.

29. Verfahren nach Anspruch 28, dadurch gekennzeichnet, daß in Arbeitsschritt c) die Wärmeübertragung über eine Wärmeübertragungswandung der Reaktionskammer in die Kammer hinein und aus der Kammer heraus erfolgt und daß die Wärmeübertragungswandung mindestens ein wärmeleitfähiges Material aufweist.

30. Verfahren nach Anspruch 29, dadurch gekennzeichnet, daß die Wärmeübertragungswandung eine Übertragungsstrecke von nicht mehr als 0,3 mm bildet und für einen Querschnittsbereich von 1 cm² einen thermischen Widerstand von nicht mehr als 5,0 °C/W besitzt.

31. Verfahren nach einem der Ansprüche 28 bis 30, dadurch gekennzeichnet, daß die Küvette in einer der mit der Reaktionskammer in Strömungsverbindung stehenden Kammern eine erste Kolben- und Zylinderanordnung besitzt, und beim Vorwärtshub des Kolbens in dieser Kammer der Druck in der Reaktionskammer steigt, und in einer anderen der Kammern, die mit der Detektionsstelle in Strömungsverbindung steht, eine zweite Kolben- und Zylinderanordnung vorgesehen ist, durch deren zweiten Kolben beim Rückhub im Zylinder eine Druckentlastung an der Detektionsstelle erfolgt, und daß in Arbeitsschritt d) der erste Kolben vorwärtsbewegt und der zweite Kolben zurückbewegt wird.

32. Verfahren nach einem der Ansprüche 28 bis 30, dadurch gekennzeichnet, daß mindestens eine Wandung der Kammern jeweils so flexibel ist, daß die Kammern durch Druck von außen zusammendrückbar und Flüssigkeit aus den Kammern übertragbar ist, und daS in Arbeitsschritt d) die flexiblen Wandungen der Kammern in einer vorbestimmten Reihenfolge von außen mit Druck beaufschlagt werden.

33. Verfahren nach einem der Ansprüche 28 bis 30, dadurch gekennzeichnet, daß die Detektionsreagenzien ein aus magnetisierbarem Material bestehendes Kügelchen enthalten, daß in den Arbeitsschritten d) bis f) die Kügelchen in die Reaktionskammer übertragen und an das amplifizierte Nukleinsäurematerial angebunden werden, das Detektionsmaterial an das amplifizierte Material angebunden wird und nicht angebundenes Detektionsmaterial durch Waschen in Gegenwart eines Magnetfeldes entfernt wird, das die Kügelchen und das angebundene Detektionsmaterial in der Reaktionskammer zurückhält.

34. Verfahren nach einem der Ansprüche 28 bis 30, dadurch gekennzeichnet, daß die Arbeitschritte d) und e) nacheinander durchgeführt werden, indem zuerst die Reaktionskammer und anschließend eine Speicherkammer mit Druck beaufschlagt wird.

35. Verfahren nach einem der Ansprüche 28 bis 30, dadurch gekennzeichnet, daß in den Arbeitschritten d) und e) die Speicherkammer und die Reaktionskammer gleichzeitig mit Druck beaufschlagt werden und die Strömung des Detektionsmaterials aufgehalten wird, bis amplifiziertes Nukleinsäurematerial übertragen worden ist.

36. Verfahren nach einem der Ansprüche 28 bis 30, daß vor Arbeitsschritt e) Detektionsmaterial, das in getrockneter Form in einer Speicherkammer vorgesehen ist, rekonstituiert wird, indem zuvor inkorporiertes Wasser aus einer Speicherkammer zu dem getrockneten Material geleitet wird.

37. Verfahren nach einem der Ansprüche 28 bis 31, dadurch gekennzeichnet, daß in Arbeitsschritt a) mindestens Blutzellen und wahlweise DNS-Extraktionsmittel in eine vorbestimmte Kammer zur Herstellung einer Lösung injiziert werden und

vor Arbeitsschritt c)

(i) DNS aus den in der vorbestimmten Kammer enthaltenen Zellen extrahiert und

(ii) nach einer geeigneten Inkubationszeit die Lösung aus extrahierter DNS und Zellfragmenten durch ein zwischen der vorbestimmten Kammer und der Reaktionskammer befindliches Filter geleitet wird, wobei das Filter so gewählt ist, daß Zellfragmente zurückgehalten werden, jedoch DNS durchgelassen wird.

## Revendications

1. Cuve à jeter (10 ; 10A ; 10B ; 100 ; 100C ; 100D ; 100E ; 100F) destinée à réaliser l'amplification et de la détection d'acide nucléique, comprenant :

   un premier compartiment (40 ; 40A ; 40B; 126C);

   une pluralité de compartiments supplémentaires (26, 30, 32, 34, 36, 38, 42 ; 61, 26A, 30A, 38A; 26B, 30B, 32B, 34B, 36B, 38B, 42B ; 126, 132, 134, 136, 138, 139, 140, 182 ; 132C, 134C, 136C, 138C ; 126D, 132D, 134D, 138D ; 126E, 136E, 138E, 182E, 250, 252, 254), au moins un des compartiments supplémentaires contenant un matériel pour amplifier et détecter l'acide nucléique;

   des moyens de connexion de fluides (44, 48, 49, 50, 52, 54 ; 62, 44A, 48A, 49A, 50A, 52A, 54A ; 54B ; 144, 149, 150, 151, 152, 154, 155, 156, 157, 185 ; 144E, 151E, 157E, 262, 264, 266 ; 157F) destinés à connecter au moins un des compartiments supplémentaires au premier compartiment (40 ; 40A ; 40B ; 126C) de façon que le matériel retenu dans le compartiment supplémentaire au nombre minimal de un soit transférable au premier compartiment (40 ; 40A ; 40B ; 126C) lorsqu'une pression est appliquée sur le matériel retenu dans ce compartiment;

   un site de détection (39 ; 190 ; 190F) destiné à immobiliser l'acide nucléique en vue de sa détection après son amplification et

   un moyen de transfert thermique permettant la soumission de matériel à un cycle actif ou passif dans la cuve;

   caractérisée en ce que le premier compartiment (40 ; 40A ;40B ; 126C) et le site de détection (39 ; 190 ; 190F) sont fermés de façon interdisant l'écoulement de fluides vers des emplacements externes à la cuve de sorte que des aérosols ne sont pas émis durant l'amplification et la détection.

2. Cuve selon la revendication 1, dans laquelle le moyen de transfert thermique comprend une paroi du premier compartiment (40 ; 40A; 40B ; 126C) qui est en un matériau thermoconducteur.

3. Cuve selon la revendication 2, dans laquelle la paroi de transfert thermique possède un chemin thermique de longueur inférieure ou égale à environ 0,3 mm et une résistance thermique, pour une aire de section transversale de 1 cm$^2$, inférieure ou égale à environ 5,0 ° C/W.

4. Cuve selon l'une quelconque des revendications précédentes, dans laquelle au moins une paroi du compartiment supplémentaire au nombre minimal de un est suffisamment souple pour permettre la compression du compartiment par une pression externe et le transfert du matériel qu'il contient au premier compartiment (40 ; 40A ; 40B ; 126C).

5. Cuve selon la revendication 4, dans laquelle les autres compartiments (26, 30, 32, 34, 36, 38, 42 ; 61, 26A, 30A, 38A ; 26B, 30B, 32B, 34B, 36B, 38B, 42B) et leurs moyens de connexion par fluides (44, 48, 49, 50, 52, 54 ; 62, 44A, 48A, 49A, 50A, 52A, 54A ; 54B) sont disposés de telle sorte que le matériel retenu dans chaque compartiment peut être expulsé dans un ordre prescrit par application d'une pression externe linéairement le long des surfaces externes de la cuve.

6. Cuve selon la revendication 4 ou 5, dans laquelle la pression externe est appliquée au moyen d'un rouleau (60) qui avance au-dessus d'une surface externe de la cuve.

7. Cuve selon l'une quelconque des revendications 4 à 6, dans laquelle un des compartiments supplémentaires (61) contient de l'eau et est connecté fluidiquement à tous les autres compartiments supplémentaires (26A, 30A, 38A), ce compartiment étant positionné de façon à être le premier à être comprimé.

8. Cuve selon l'une quelconque des revendications 1 à 3, dans laquelle un des compartiments supplémentaires est un compartiment réactionnel (26 ; 26A ; 26B ; 126 ;126C ; 126D ; 126E) dans lequel est introduit l'acide nucléique.

9. Cuve selon la revendication 8, dans laquelle le compartiment réactionnel (26 ; 26A ; 26B ; 126 ; 126C ; 126D ; 126E) contient des agents amplificateurs (28) préincorporés n'ayant pas réagi,.

10. Cuve selon la revendication 9, dans laquelle les agents amplificateurs n'ayant pas réagi sont l'enzyme polymérase, des acides nucléiques amorces et des nucléotides.

11. Cuve selon la revendication 9, dans laquelle les agents amplificateurs n'ayant pas réagi sont de la polymérase TAQ, des acides nucléiques amorces et des nucléotides.

**12.** Cuve selon l'une quelconque des revendications 1 à 7, comprenant en outre un moyen de compression destiné à comprimer le matériel retenu dans le compartiment supplémentaire au nombre minimal de un, pour effectuer le transfert de ce matériel au premier compartiment.

**13.** Cuve selon la revendication 12, dans laquelle le moyen de compression comprend un premier dispositif piston-cylindre (113, 115) logé à l'intérieur du compartiment supplémentaire au nombre minimal de un.

**14.** Cuve selon la revendication 12 ou 13, dans laquelle le moyen de compression comprend un second dispositif piston-cylindre (182, 184 ; 182C, 184C ; 182E, 184E) qui est connecté fluidiquement au premier compartiment (140 ; 126C) de sorte que lorsque le second piston (184 ; 184C ; 184E) est en arrière dans son cylindre, il réduit la pression dans le premier compartiment.

**15.** Cuve selon l'une quelconque des revendications 12 à 14, dans laquelle un des compartiments supplémentaires est un compartiment réactionnel (126D) dans lequel l'acide nucléique est introduit, la cuve comprenant en outre un compartiment d'extraction (200) positionné fluidiquement en amont du compartiment réactionnel (126D) et un filtre (208) interposé dans le passage pour fluides entre le compartiment d'extraction (200) et le compartiment réactionnel (126D), le compartiment d'extraction (200) permettant de séparer l'ADN d'au moins des globules sanguins, les fragments cellulaires étant séparés de l'ADN par le filtre (208), de sorte que seul l'ADN se dirige vers le compartiment réactionnel (126D).

**16.** Cuve selon l'une quelconque des revendications précédentes, dans laquelle le site de détection (39 ; 190 ; 190F) comprend une sonde contenant un matériel génétique complémentaire d'un ADN et qui par conséquent s'hybride avec cet ADN sous chauffage approprié.

**17.** Cuve selon la revendication 16, dans laquelle le site de détection comprend en outre une sonde témoin positif contenant un matériel génétique complémentaire d'un ADN amplifié toujours présent dans un échantillon sanguin.

**18.** Cuve selon la revendication 17, dans laquelle le matériel génétique constituant la sonde témoin positif est complémentaire de $\beta$-globine.

**19.** Cuve selon la revendication 17 ou 18, dans laquelle la sonde témoin positif est immobilisée sur une partie du site de détection, en l'absence d'ADN amplifié.

**20.** Cuve selon l'une quelconque des revendications 16 à 19, dans laquelle le site de détection comprend en outre une sonde témoin négatif contenant un matériel génétique qui n'est pas complémentaire d'un ADN quelconque supposé être présent après l'amplification d'un échantillon sanguin.

**21.** Cuve selon la revendication 20, dans laquelle la sonde témoin négatif est immobilisée sur une partie du site de détection, en l'absence d'ADN amplifié.

**22.** Cuve selon la revendication 20 ou 21, dans laquelle la sonde témoin positif et la sonde témoin négatif sont immobilisées sur une partie du site de détection en l'absence d'ADN amplifié quelconque, chacune des parties immobilisées du site étant sensiblement exempte de l'autre sonde témoin.

**23.** Cuve selon l'une quelconque des revendications précédentes, dans laquelle le site de détection (39) comprend une feuille support (41) sur laquelle sont disposées une pluralité de piles (43, 43', 43'', 43'''), chaque pile comprenant une bille de polymère à laquelle est fixée une sonde d'ADN.

**24.** Cuve selon l'une quelconque des revendications 1 à 22, dans laquelle le site de détection (39) comprend une feuille support (41) sur laquelle sont disposées une pluralité de piles (43, 43', 43'', 43'''), chaque pile comprenant une bille de polymère à laquelle est fixée de l'avidine.

**25.** Cuve selon l'une quelconque des revendications 16 à 22, dans laquelle le site de détection se trouve dans le premier compartiment (126C) et le matériel destiné à la détection de l'acide nucléique comprend une bille comprenant un matériau qu'il est possible d'aimanter.

**26.** Appareil d'amplification et de détection d'ADN, comprenant une cuve selon l'une quelconque des revendications précédentes.

**27.** Appareil d'amplification et de détection d'ADN, comprenant une cuve contenant

    i) une pluralité de compartiments et des moyens d'interconnexion de chacun d'entre eux à au moins un autre compartiment, les compartiments comprenant a) au moins un compartiment réactionnel destiné à l'amplification de brins d'ADN, b) au moins un compartiment de détection destiné à détecter l'ADN amplifié et comprenant un site de détection et c) un moyen d'apport d'un matériel de détection aux brins d'ADN amplifié ;

ii) un moyen permettant la soumission à un cycle thermique actif ou passif du contenu du compartiment réactionnel dans une plage allant d'environ 30°C à environ 95°C et iii) un moyen d'accès de liquide connecté uniquement au compartiment réactionnel au nombre minimal de un, destiné à permettre l'injection dans le compartiment réactionnel d'un ADN échantillon en vue de son amplification caractérisé en ce que la cuve comprend en outre

iv) un moyen de scellement de la cuve empêchant le passage d'ADN après l'injection de l'ADN échantillon ;

et l'appareil contenant en outre un moyen permettant d'envoyer au moins le matériel de détection et un brin d'ADN dans le compartiment de détection et sur le site de détection ;

de telle sorte qu'une fois l'échantillon d'ADN injecté dans les compartiments et l'ouverture d'accès fermée, les contenus fluidiques des compartiments sont enfermés de façon interdisant tout contact avec le manipulateur et l'environnement durant la réaction entière d'amplification et de détection.

28. Procédé d'amplification et de détection d'acide nucléique dans une cuve fermée, comprenant les étapes consistant à

a) injecter un échantillon d'acide nucléique dans une cuve qui comprend une pluralité de compartiments, dont un compartiment réactionnel dans lequel sont présents des réactifs amplificateurs, et un compartiment de stockage à utiliser avec un matériel de détection, au moins un des compartiments comprenant un site de détection, et un moyen d'interconnexion des compartiments pour assurer le transfert de fluides ;

b) fermer hermétiquement à demeure les parties de la cuve contenant l'acide nucléique pour verrouiller tout l'acide nucléique dans la cuve;

c) amplifier l'acide nucléique par soumission de la cuve à un cycle de variations thermiques présélectionnées pour rendre les réactifs efficaces;

d) transférer fluidiquement l'acide nucléique amplifié du compartiment réactionnel au site de détection;

e) transférer fluidiquement le matériel de détection au site de détection et

f) détecter l'acide nucléique amplifié au site de détection à l'aide du matériel de détection, l'acide nucléique demeurant confiné à l'intérieur de la cuve.

29. Procédé selon la revendication 28, dans lequel l'étape c) comprend l'étape supplémentaire consistant à transférer de la chaleur à travers une paroi de transfert thermique du compartiment réactionnel, à l'intérieur et hors du compartiment, la paroi de transfert thermique comprenant au moins un matériau thermoconducteur.

30. Procédé selon la revendication 29, dans lequel la paroi de transfert thermique a un chemin thermique de longueur inférieure ou égale à environ 0,3 mm et une résistance thermique, pour une aire dc section transversale de 1 cm$^2$, inférieure ou égale à environ 5,0°C/W.

31. Procédé selon l'une quelconque des revendications 28 à 30, dans lequel la cuve comprend en outre un premier dispositif piston-cylindre dans l'un des compartiments qui est connecté fluidiquement au compartiment réactionnel de telle sorte que l'avance du piston dans le compartiment élève la pression dans le compartiment réactionnel, et un second dispositif piston-cylindre dans un autre des compartiments qui est connecté fluidiquement au site de détection de telle sorte que, lorsque le second piston est en arrière dans son cylindre, il réduit la pression au site de détection et l'étape d) comprend l'étape consistant à faire avancer le premier piston tout en ramenant le second piston vers l'arrière.

32. Procédé selon l'une quelconque des revendications 28 à 30, dans lequel au moins une paroi des compartiments est suffisamment souple pour permettre la compression des compartiments par une pression externe pour provoquer le transfert des liquides hors des compartiments et dans lequel l'étape d) comprend l'étape consistant à appliquer une pression externe sur les parois souples des compartiments dans un ordre prescrit.

33. Procédé selon l'une quelconque des revendications 28 à 30, dans lequel les réactifs de détection comprennent une bille comprenant un matériau qu'il est possible d'aimanter et dans lequel les étapes d) à f) comprennent le transfert des billes au compartiment réactionnel, la fixation des billes à l'acide nucléique amplifié, la fixation du matériel de détection à l'acide nucléique amplifié et la séparation par lavage du matériel de détection non fixé en présence d'un champ magnétique qui retient les billes et le matériel de détection fixé à l'intérieur du compartiment réactionnel.

34. Procédé selon l'une quelconque des revendications 28 à 30, dans lequel les étapes d) et e) sont mises en oeuvre successivement, par compression du compartiment réactionnel en premier lieu, puis d'un compartiment de stockage.

35. Procédé selon l'une quelconque des revendications 28 à 30, dans lequel les étapes d) et e) sont mises en oeuvre par compression simultanée du compartiment de stockage et du compartiment réactionnel et retard de l'écoulement du matériel de détection jusqu'après le transfert de l'acide nucléique amplifié.

36. Procédé selon l'une quelconque des revendications 28 à 30, et comprenant en outre comme étape précédant l'étape e) l'étape consistant à reconstituer le matériel de détection déposé sous forme séchée dans un compartiment de stockage, par transfert à la substance séchée d'eau préincorporée dans un compartiment de stockage.

37. Procédé selon l'une quelconque des revendications 28 à 31, dans lequel l'étape a) comprend l'étape consistant à injecter au moins des globules sanguins et éventuellement des agents d'extraction d'ADN dans un compartiment prédéterminé pour former une solution ;
et comprenant en outre avant l'étape c), les étapes consistant à :
i) extraire l'ADN des globules dans le compartiment prédéterminé et
ii) après une période d'incubation appropriée, envoyer la solution d'ADN extrait et de fragments cellulaires sur un filtre disposé entre le compartiment prédéterminé et le compartiment réactionnel, le filtre étant calibré pour retenir les fragments cellulaires et laisser passer l'ADN.

FIG. 1

FIG. 2

FIG. 3

FIG. 4

FIG. 5

FIG. 6

FIG. 8

FIG. 7

EP 0 381 501 B1

FIG. 9

*100*

*121*  *123*  *190*  *194*

*151*  *114*  *126*  *157*  *196*  *192*

# FIG. 10

*100*

*113*  *113*  *113*  *113*  *115*  *184*

IX ↓                                        ↓ IX

# FIG. 11

*100*

*149*  *150*  *154*  *152*  *155*  *156*

*151*  *126*  *144*  *114*  *185*

# FIG. 12

FIG. 13

EP 0 381 501 B1

FIG. 14

FIG. 19

FIG. 15

FIG. 16

FIG. 17

FIG. 18